(19) **Europäisches Patentamt / European Patent Office / Office européen des brevets**

(11) **EP 4 159 758 A1**

(12) **EUROPEAN PATENT APPLICATION**
published in accordance with Art. 153(4) EPC

(43) Date of publication:
**05.04.2023 Bulletin 2023/14**

(21) Application number: **21814253.7**

(22) Date of filing: **27.05.2021**

(51) International Patent Classification (IPC):
**C07K 16/22** (2006.01)    **C07K 14/50** (2006.01)
**A61K 38/00** (2006.01)    **A61P 3/10** (2006.01)

(52) Cooperative Patent Classification (CPC):
**A61K 38/00; A61K 38/18; A61K 47/64; A61P 1/16;**
**A61P 3/00; A61P 3/04; A61P 3/06; A61P 3/10;**
**A61P 9/00; A61P 13/12; A61P 25/00; C07K 14/50;**
**C07K 16/22; C07K 19/00; C12N 15/62;**   (Cont.)

(86) International application number:
**PCT/CN2021/096292**

(87) International publication number:
**WO 2021/239046 (02.12.2021 Gazette 2021/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2020 CN 202010468160**

(71) Applicant: **Jiangsu Kanionreal Biomedical Technology Co., Ltd.**
**Lianyungang, Jiangsu 222047 (CN)**

(72) Inventors:
• **XIAO, Wei**
  **Lianyungang, Jiangsu 222047 (CN)**
• **LI, Deshan**
  **Lianyungang, Jiangsu 222047 (CN)**

• **YU, Dan**
  **Lianyungang, Jiangsu 222047 (CN)**
• **LIU, Zhihang**
  **Lianyungang, Jiangsu 222047 (CN)**
• **WANG, Zhenzhong**
  **Lianyungang, Jiangsu 222047 (CN)**
• **YIN, Chengkai**
  **Lianyungang, Jiangsu 222047 (CN)**
• **SUN, Xu**
  **Lianyungang, Jiangsu 222047 (CN)**

(74) Representative: **WSL Patentanwälte Partnerschaft mbB**
**Kaiser-Friedrich-Ring 98**
**65185 Wiesbaden (DE)**

Remarks:
The complete document including Reference Table(s) and the Sequence Listing(s) can be downloaded from the EPO website

(54) **RHFGF21 FUSION PROTEIN, POLYNUCLEOTIDE ENCODING RHFGF21 FUSION PROTEIN, COMPOSITION CONTAINING RHFGF21 FUSION PROTEIN, AND USE OF RHFGF21 FUSION PROTEIN**

(57)    A recombinant human fibroblast growth factor 21 (rhFGF21) fusion protein, a polynucleotide encoding the rhFGF21 fusion protein, a recombinant expression vector, a recombinant cell, a pharmaceutical composition and a kit containing the rhFGF21 fusion protein, a use of the rhFGF21 fusion protein, and a method for producing the rhFGF21 fusion protein. Efficient and soluble expression is achieved using a genetic engineering method to obtain a rhFGF21-$(VPGXG)_n$ fusion protein, wherein by means of fusion of rhFGF21 and an elastin-like protein $(VPGXG)_n$ having an appropriate cycle number n, the resulting fusion protein has better biological activity than wild-type rhFGF21. Moreover, experimental results indicate that the rhFGF21-$(VPGXG)_n$ fusion protein has high expression efficiency, and can effectively reduce the blood sugar level in the body of a diabetic animal. In addition, compared with wild-type hFGF21, the fusion protein also has the advantage of a long efficacy duration in reducing the blood sugar level.

(52) Cooperative Patent Classification (CPC): (Cont.)
     **C12N 15/70; C12N 15/74**

**Description**

**FIELD OF THE INVENTION**

The present application belongs to the field of biomedicine, and particularly relates to a fusion protein comprising a recombinant human fibroblast growth factor 21 (rhFGF21) and an elastin-like protein $(VPGXG)_n$ which are fused together, a polynucleotide encoding the same, a recombinant expression vector and a recombinant cell, a composition comprising the fusion protein and a kit, use thereof, and a method of producing the fusion protein.

**BACKGROUND OF THE INVENTION**

Fibroblast growth factor 21 (FGF21) is a metabolic regulating factor produced primarily by the liver that exerts potent hypoglycemic and lipid-lowering effects in animal models of obesity and type 2 diabetes mellitus. The principal sites of metabolic actions of FGF21 are adipose tissue, liver and pancreas. Experimental studies have shown improvements in diabetes compensation and dyslipidemia after FGF21 administration in diabetic mice and primates.

However, FGF21 has a very short circulatory half-life in vivo, and thus cannot achieve the desired efficacy. Modification of FGF21 with polyethylene glycol (PEG) can effectively improve its pharmacokinetics, improve drug distribution, and enhance its efficacy. Nevertheless, current PEGylated FGF21 has drawbacks such as low reaction yield and difficulties in controlling the binding site and coupling stoichiometry, which increase the cost in production. Therefore, developing a site-specific modification method, which has mild reaction condition and simple procedure and is fast and efficient, is particularly important for ensuring the efficacy of FGF21 . Pentapeptide repeating sequence unit is a polypeptide with elastic function and temperature sensitivity which consists of five amino acids VPGXG. X (i.e., Xaa) can be any amino acid other than proline. It has good biocompatibility, but for different proteins, different cycle numbers (i.e., repetitions of VPGXG unit) will have different effects on the expression levels, half-lives, and activities. The cycle number of elastin that is appropriate for ensuring the activity of FGF21 and extending the half-life thereof has not been reported yet, and needs to be explored experimentally. Therefore, the development of a suitable fusion protein is needed to ensure the activity of human fibroblast growth factor 21 (hFGF21) and extend its half-life. Meanwhile, further development of an in vitro expression system suitable for the fusion protein is also needed to efficiently obtain an active fusion protein.

**SUMMARY OF THE INVENTION**

In this application, in view of the problems in the medicinal process of rhFGF21 , such as the short half-life and the inability to guarantee activity of rhFGF21 , the inventors provide a new recombinant human fibroblast growth factor 21 fusion protein via research. Further, the inventors also develop a prokaryotic expression system suitable for the fusion protein, thus enabling soluble expression of the rhFGF21 fusion protein with desired efficiency.

In one aspect, the present application relates to a recombinant human fibroblast growth factor 21 fusion protein, wherein the fusion protein comprises the rhFGF21 and $(VPGXG)_n$ which are fused together, the rhFGF21 comprises an amino acid sequence set forth in SEQ ID NO: 1 or an amino acid sequence with at least 80% identity to SEQ ID NO: 1; X is A and/or V; and n is an integer selected from 20-80.

In another aspect, the present application relates to a polynucleotide encoding the above-mentioned fusion protein.

In another aspect, the present application relates to a recombinant expression vector containing the polynucleotide encoding the above-mentioned fusion protein.

In another aspect, the present application relates to a recombinant cell containing the above-mentioned recombinant expression vector or having the above-mentioned polynucleotide integrated in its genome.

In another aspect, the present application relates to a pharmaceutical composition, wherein the pharmaceutical composition comprises the above-mentioned fusion protein.

In another aspect, the present application relates to a kit, wherein the kit comprises the above-mentioned fusion protein or the above-mentioned pharmaceutical composition.

In another aspect, the present application relates to use of the above-mentioned fusion protein in the preparation of a medicament for the prevention or treatment of a disease associated with abnormal glucose metabolism. Alternatively, the present application relates to a method of preventing or treating a disease associated with abnormal glucose metabolism, comprising administering to a subject in need thereof the above-mentioned fusion protein. Alternatively, the present application relates to a fusion protein for use in the prevention or treatment of a disease associated with abnormal glucose metabolism.

In yet another aspect, the present application relates to a method of producing the above-described fusion protein of the present application, wherein the method comprises: culturing the above-mentioned recombinant cell, then inducing the recombinant cell to express and purifying the expression product to obtain the fusion protein.

Herein, the effect of the fusion protein described in the present application on the treatment of diabetes is evaluated using diabetic model murine. The result of the animal experiments shows that the fusion protein of the present application can effectively reduce the blood glucose level in the diabetic model animal, and has the advantages such as a longer efficacy duration (i.e., longer half-life) in reducing the blood glucose level, compared to wild-type hFGF21. Also, the fusion protein described in the present application exhibits good ability in promoting glucose uptake, and can achieve the desired expression efficiency.

**DETAILED DESCRIPTION OF THE DRAWINGS**

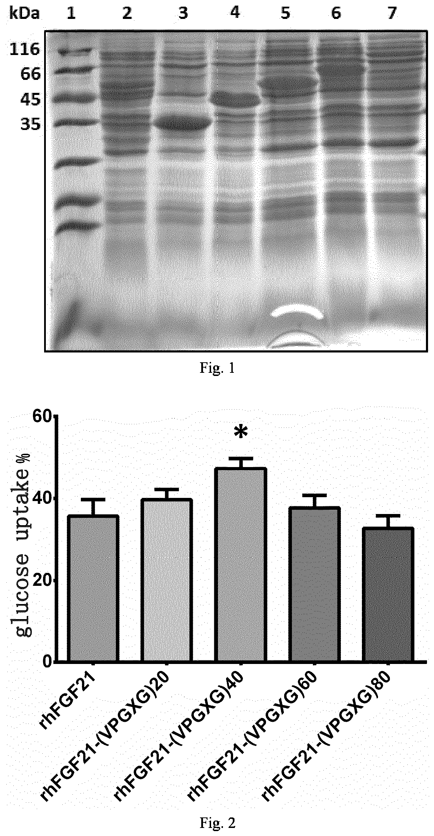

Figure 1 is the electropherogram showing the expression amount of SUMO-rhFGF21-$(VPGXG)_n$ fusion protein, wherein the reference signs each represent the follows: 1 : protein marker; 2: protein expression before IPTG induction; 3: SUMO-rhFGF21 ; 4: SUMO-rhFGF21-$(VPGXG)_{20}$; 5: SUMO-rhFGF21-$(VPGXG)_{40}$; 6: SUMO-rhFGF21-$(VPGXG)_{60}$; 7: SUMO-rhFGF21-$(VPGXG)_{80}$. Gray-scale analysis of the results shows that the percentage of SUMO-rhFGF21-$(VPGXG)_{40}$ expression amount relative to the total protein expression amount is higher than the other groups, i.e., under the same conditions, SUMO-rhFGF21-$(VPGXG)_{40}$ is more efficiently expressed: SUMO-rhFGF21-$(VPGXG)_{40}$, 11.2%; SUMO-rhFGF21-$(VPGXG)_{20}$, 9.7%; SUMO-rhFGF21-$(VPGXG)_{60}$, 10.1%; SUMO-rhFGF21-$(VPGXG)_{80}$, 3.2%.

Figure 2 is a graph showing the changes in glucose uptakes of β-Klotho-3T3-L1 adipocytes after being stimulated with different test samples. After being stimulated with 10 nmol/L SUMO-rhFGF21 (shown as "rhFGF21"), the glucose uptake level of β-Klotho-3T3-L1 adipocytes was about 35%; after being stimulated with 10 nmol/L rhFGF21-$(VPGXG)_{20}$, the glucose uptake level of β-Klotho-3T3-L1 adipocytes increased to about 39%; after being stimulated with 10 nmol/L rhFGF21-$(VPGXG)_{40}$, the glucose uptake level of β-Klotho-3T3-L1 adipocytes increased to about 46%;

after being stimulated with 10 nmol/L rhFGF21-$(VPGXG)_{60}$, the glucose uptake level of β-Klotho-3T3-L1 adipocytes increased to about 37%; and after being stimulated with 10 nmol/L rhFGF21 -$(VPGXG)_{80}$, the glucose uptake level of β-Klotho-3T3-L1 adipocytes increased to about 32%. It can be seen that 10 nmol/L rhFGF21-$(VPGXG)_{40}$ has a better ability to promote glucose uptake at cellular level compared to the other groups.

Figure 3 is a HPLC spectrogram showing the analysis of the purity of rhFGF21-$(VPGXG)_{40}$ protein. Through the HPLC analysis, the peak of rhFGF21-$(VPGXG)_{40}$ protein started to appear at a retention time of about 6 min, and its purity can reach 95% or more.

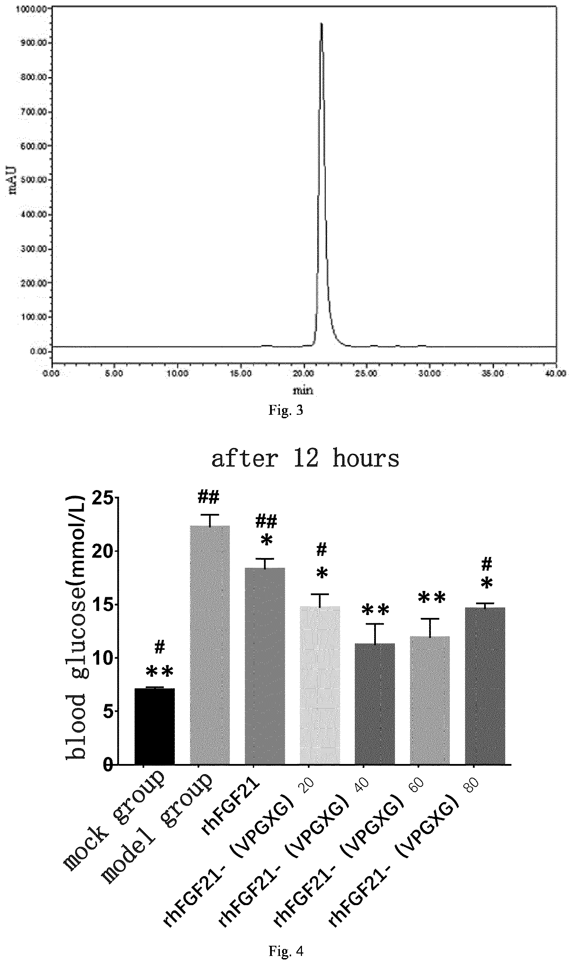

Figure 4 is a graph showing the hypoglycemic results after treating the STZ model mice with different test samples for 12h, wherein the hypoglycemic effect of rhFGF21 treatment group gradually decreased after 12 h of administration compared to the model group; rhFGF21-$(VPGXG)_{20}$ treatment group, rhFGF21-$(VPGXG)_{40}$ treatment group, rhFGF21-$(VPGXG)_{60}$ treatment group and rhFGF21-$(VPGXG)_{80}$ treatment group still showed certain therapeutic effects, and the rhFGF21-$(VPGXG)_{40}$ treatment group showed the best hypoglycemic effect, wherein $*p<0.05$ and $**p<0.01$, compared to the model group; $^{\#}p<0.05$ and $^{\#\#}p<0.01$, compared to the normal group.

Figure 5 is a graph showing the results of the oral glucose tolerance test (OGTT).

After 30 min of oral administration of glucose, the blood glucose level of the model group increased significantly, and the blood glucose level of the rhFGF21-$(VPGXG)_{40}$ treatment group was significantly lower than that of the model group, wherein $*p<0.05$, $**p<0.01$, compared to the model group; $^{\#}p<0.05$, $^{\#\#}p<0.01$, compared to the normal group.

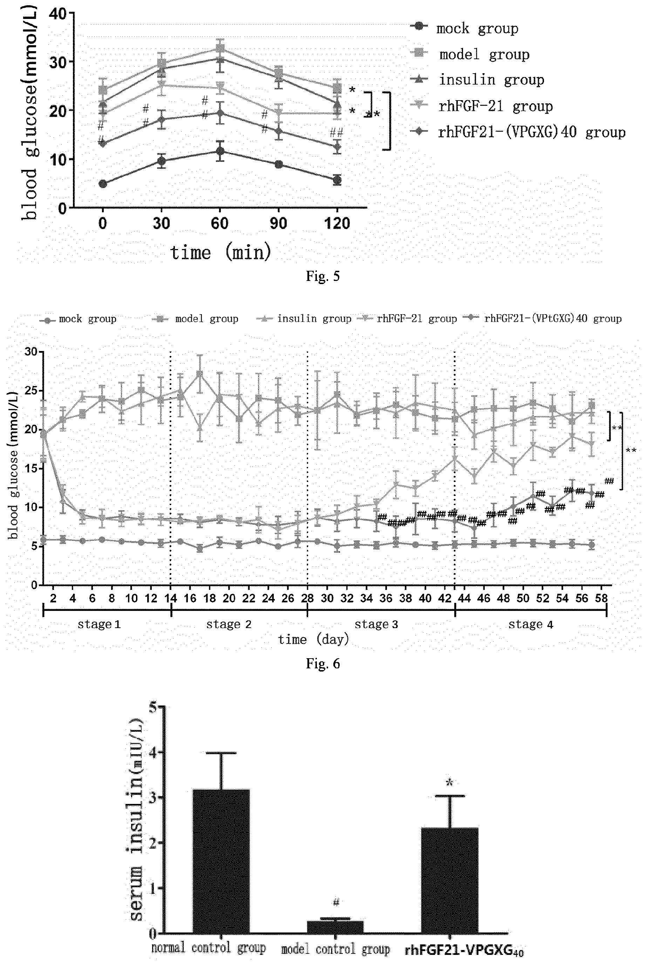

Figure 6 is a graph showing the changes in blood glucose of mice in each group over 58 days. The blood glucose of mice in the model group always maintained at a relatively high level; while the blood glucose of mice in the rhFGF21-$(VPGXG)_{40}$ treatment group decreased significantly compared to the model group, and the blood glucose thereof maintained at a relative low level and approached that of the mice in the normal group after treatment, and the efficacy thereof lasted longer than that of rhFGF21, wherein $*p<0.05$, $**p<0.01$, compared to the model group; and $^{\#}p<0.05$, $^{\#\#}p<0.01$, compared to the normal group.

Figure 7 is a graph showing the serum insulin concentration of mice in each group. The serum insulin concentration in the model group mice significantly decreased, while the serum insulin concentration of mice in the rhFGF21-(VPGXG)$_{40}$ treatment group significantly increased compared to the model group, wherein *$p<0.05$, **$p<0.01$, compared to the model group; and #$p<0.05$, ##$p<0.01$, compared to the normal group.

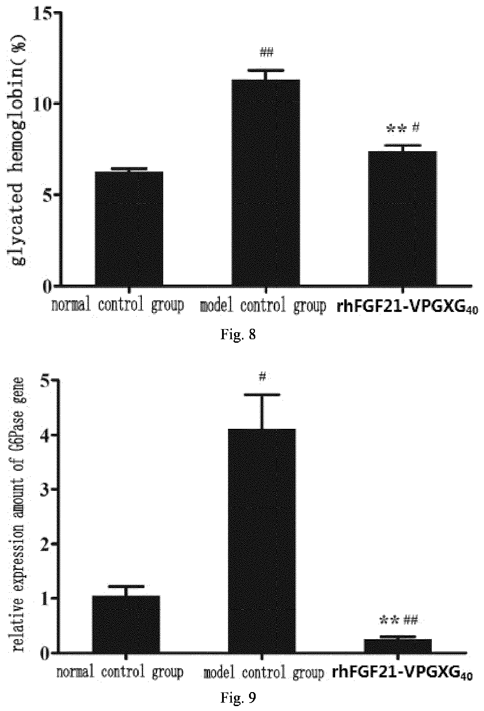

Figure 8 is a graph showing the glycated hemoglobin level in each group of mice. After administration of the corresponding test samples for 30 days, the glycated hemoglobin level in the model group mice maintained at a relatively high level, while the rhFGF21-(VPGXG)$_{40}$ treatment group mice showed a significant decrease in glycated hemoglobin levels, wherein *$p<0.05$, **$p<0.01$, compared to the model group; and #$p<0.05$, ##$p<0.01$ , compared to the normal group.

Figure 9 is a graph showing the relative expression amount of G6Pase gene in the livers of mice in each group. The G6Pase expression amount in the livers significantly increased in the model group mice, while the G6Pase expression amount in the livers significantly decreased in the rhFGF21-(VPGXG)$_{40}$ treatment group mice, wherein *$p<0.05$, **$p<0.01$, compared to the model group; #$p<0.05$, ##$p<0.01$, compared to the normal group.

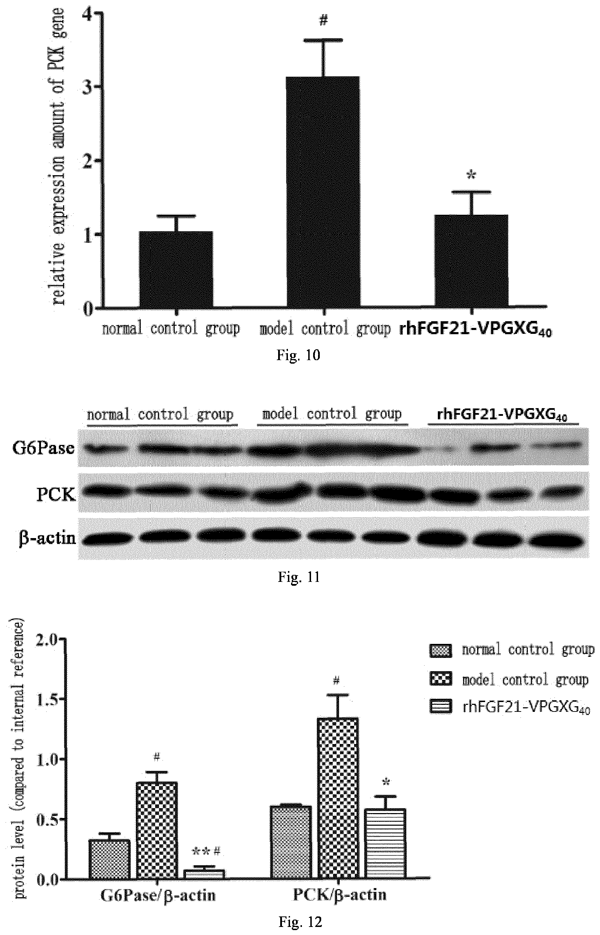

Figure 10 is a graph showing the relative expression amount of PCK gene in the livers of mice of each group. The PCK gene expression amount in the livers significantly increased in the model group mice, while the PCK gene expression amount in the livers significantly decreased in the rhFGF21-(VPGXG)$_{40}$ treatment group mice, wherein *$p<0.05$, **$p<0.01$, compared to the model group; and #$p<0.05$, ##$p<0.01$, compared to the normal group.

Figure 11 and Figure 12 are graphs showing the expression amounts of G6Pase and PCK proteins in the livers of mice in each group. The expression amounts of G6Pase and PCK proteins in the livers significantly increased in the model group mice, while the expression amounts of G6Pase and PCK proteins in the livers significantly decreased in the rhFGF21-(VPGXG)$_{40}$ treatment mice, wherein *$p<0.05$, **$p<0.01$, compared to the model group; and #$p<0.05$, ##$p<0.01$, compared to the normal group.

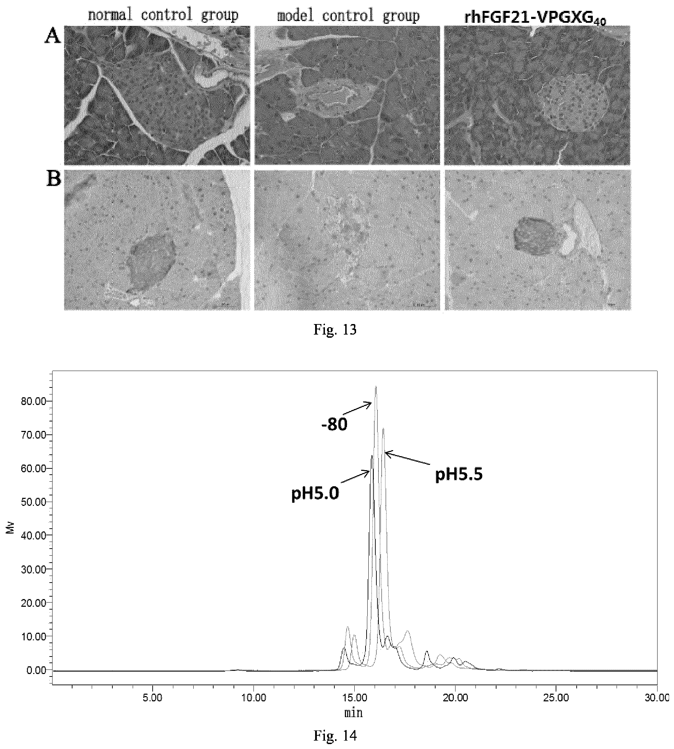

Figure 13 is a picture showing the results of the HE staining and immunohistochemistry of pancreas. Pancreatic islets of model group mice were damaged more severely, and the amount of the secreted insulin significantly decreased, while the islets of rhFGF21-(VPGXG)$_{40}$ treatment group mice significantly improved compared to the model group.

Figure 14 is a spectrogram showing the HPLC analysis result of rhFGF21-(VPGXG)$_{40}$ protein after being stored in citrate buffer solutions with different pH values (pH 5.0 and 5.5) for 14 days at 25°C and the same protein stored at -80°C.

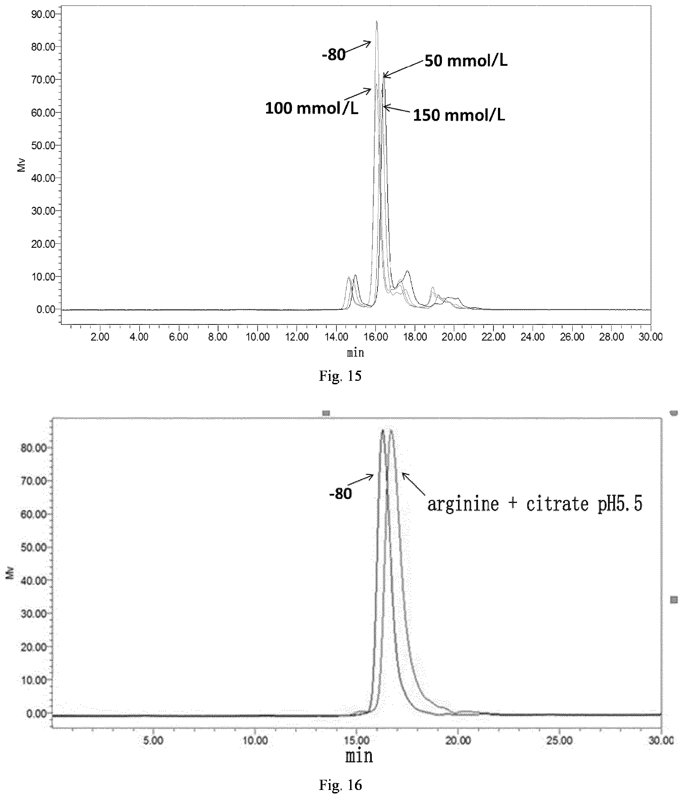

Figure 15 is a spectrogram showing the HPLC analysis result of rhFGF21-(VPGXG)$_{40}$ protein after being stored in aqueous arginine solutions with different concentrations (including 50 mM, 100 mM and 150 mM arginine) for 25 days at 25°C and the same protein stored at -80°C.

Figure 16 is a spectrogram showing the HPLC analysis result of rhFGF21-(VPGXG)$_{40}$ protein after being stored in arginine + citrate buffer solution (pH 5.5) for 35 days at 25°C and the same protein stored at -80°C.

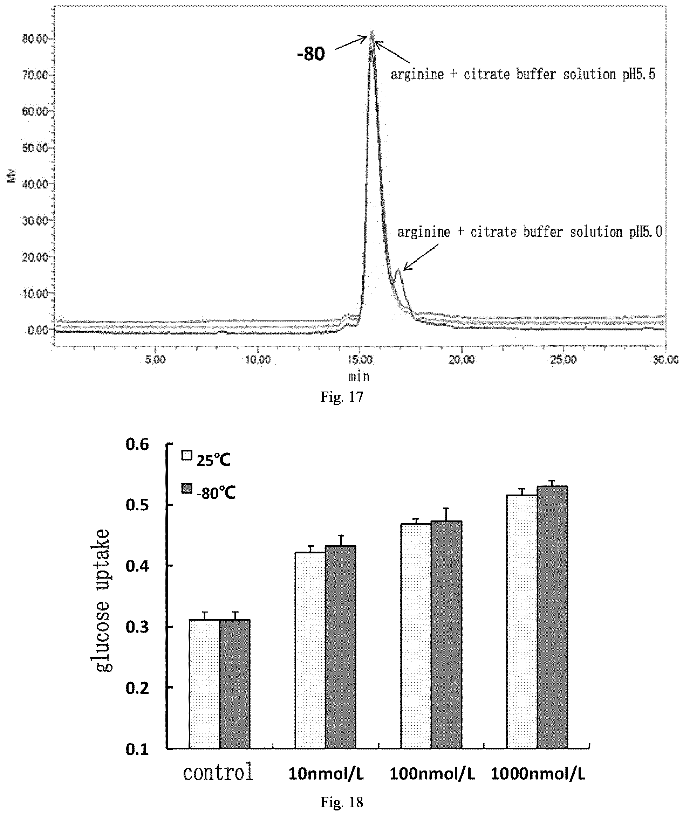

Figure 17 is a spectrogram showing the HPLC analysis result of rhFGF21-(VPGXG)$_{40}$ protein after being stored in arginine + citrate buffer solutions having different pH values (pH 5.0 and 5.5) for 60 days at 4°C and the same protein stored at -80°C.

Figure 18 is a graph showing the result of promoting the glucose uptake of HepG2 cells with different doses (10 nmol, 100 nmol and 1000 nmol) of rhFGF21-(VPGXG)$_{40}$ protein after being stored at 25°C or -80°C for 35 days.

In the fusion proteins involved in each of the above figures, X is A and V, and the ratio of A to V is 2:3.

**EMBODIMENTS OF THE INVENTION**

Hereinafter, the present application is further described in combination with particular embodiments, and the advantages and features of the present application will become clearer with description. However, these embodiments are merely exemplary and do not constitute any limitation to the scope of the present application. It should be understood by the person skilled in the art that although modifications or substitutions may be made to the details and form of the technical solutions of the present application without departing from its spirit and scope, such modifications and substitutions fall within the protection scope of the present application.

Unless defined otherwise, the technical and scientific terms used herein have the same meanings as commonly understood by the person of ordinary skill in the art to which this disclosure belongs, which can be seen in, for example, Singleton et al., Dictionary of Microbiology and Molecular Biology 2nd ed., J. Wiley & Sons (New York, NY 1994); Sambrook et al., Molecular Cloning, A Laboratory Manual, Cold Springs Harbor Press (Cold Springs Harbor, NY 1989).

In the present disclosure, unless stated otherwise, the terms "model group" and "model control group" can be used interchangeably, and the terms "normal group", "mock group", and "normal control group" can be used interchangeably.

Unless stated otherwise, the terms "recombinant plasmid" and "recombinant expression vector" herein can be used interchangeably.

Unless stated otherwise, the term "linker" herein can be an amino acid sequence (which may be, for example, from about 1 to about 5 amino acids in length) that is used to link rhFGF21 and $(VPGXG)_n$ as well as the repeating units in $(VPGXG)_n$ together, and can be used in the identification, which may include, for example, a linker peptide and a restriction site common known in the art, but are not limited thereto.

Unless stated otherwise, a singular term herein encompasses the plural form thereof, and *vice versa*.

Unless stated otherwise, the terms "comprise", "comprises" and "comprising" or equivalents thereof ("contain", "containing", "include" and "including") herein are open-ended mode expressions which mean that other unspecified elements, components and steps may be included in addition to those already listed.

Unless stated otherwise, the term "treatment" herein includes reversing, reducing, alleviating, ameliorating, suppressing, slowing down or stopping the progression or severity of the related condition.

Unless stated otherwise, the term "subject" herein can be used interchangeably with the terms "individual" and "patient", and includes vertebrates, such as birds, fish, mammals, such as, but is not limited to, mice, rats, guinea pigs, dogs, pigs, chickens, rabbits, monkeys (e.g., rhesus monkeys), humans, etc.

Unless stated otherwise, all the numbers used herein to express amounts of ingredients, measured values, or reaction conditions should be understood as being modified in all cases by the term "about". When being attached to a percentage, the term "about" can represent ±1%.

In the present disclosure, the percentage of identity (degree of homology) between sequences can be determined by comparing two sequences using, for example, freely accessible computer programs in the World Wide Web that are commonly used for this purpose (e.g., BLASTp or BLASTn with default settings).

In one embodiment, the present application provides a recombinant human fibroblast growth factor 21 fusion protein, wherein the fusion protein comprises rhFGF21 and $(VPGXG)_n$ which are fused together, the rhFGF21 comprises an amino acid sequence set forth in SEQ ID NO: 1 or an amino acid sequence with at least 80% identity to SEQ ID NO: 1; X is A and/or V; and n is an integer selected from 20-80.

In a preferred embodiment, the rhFGF21 comprises an amino acid sequence with at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, 98%, or 99% identity to SEQ ID NO: 1.

In a preferred embodiment, the rhFGF21 has an amino acid sequence set forth in SEQ ID NO: 1 (SEQ ID NO: 1:

HPIPDSSPLLQFGGQVRQRYLYTDDAQQTEAHLEIREDGTVGGAADQSPESLLQLKALKPGVIQILGVKTS
RFLCQRPDGALYGSLHFDPEACSFRELLLEDGYNVYQSEAHGLPLHLPGNKSPHRDPAPRGPARFLPLPGL
PPALPEPPGILAPQPPDVGSSDPLSMVGPSQGRSPSYAS）.

In a preferred embodiment, X is A and V, and the ratio of A to V is (1-3):(1-3), for example, the ratio of A to V is (1-2):(1-3), (1-2):(2-3), (2-3):(1-3), (2-3):(2-3), (2-3):(1-2),1:(1-3),2:(1-3), or 3:(1-3). In a more preferred embodiment, X is A and V, and the ratio of A to V is 2:3 (i.e., $(VPGXG)_n$ is now $[(VPGAG)_2(VPGVG)_3]_m$, where the value of m meets with n=mx(2+3)).

In a preferred embodiment, n is an integer selected from 30-80, for example, n is an integer selected from 30-70, 30-60, 30-50, 40-80, 40-70, 40-60, 40-50. In some particular embodiments, n is 20, 25, 30, 35, 40, 45, 50, 55, 60, 65, 70, or 75. In some particular embodiments, n is 30, 31, 32, 33, 34, 35, 36, 37, 38, 39, 40, 41, 42, 43, 44, 45, 46, 47, 48, 49, 50, 51 52, 53, 54, 55, 56, 57, 58, 59, 60, 61, 62, 63, 64, 65, 66, 67, 68, 69, or 70. In some more preferred embodiments, n is 40-60, such as 40.

In a preferred embodiment, the fusion protein consists of rhFGF21, $(VPGXG)_n$, and an optional linker, which are fused together.

In a preferred embodiment, the amino acid sequence of the linker is RS and/or GS.

In a preferred embodiment, the fusion protein has an amino acid sequence set forth in SEQ ID NO: 2 or an amino acid sequence with at least 80%, e.g., at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, 98%, or 99% identity to SEQ ID NO: 2.

In a preferred embodiment, the fusion protein has an amino acid sequence set forth in SEQ ID NO: 2 (SEQ ID NO: 2:

```
HPIPDSSPLLQFGGQVRQRYLYTDDAQQTEAHLEIREDGTVGGAADQSPESLLQLKALKPGVIQILGVKTS
RFLCQRPDGALYGSLHFDPEACSFRELLLEDGYNVYQSEAHGLPLHLPGNKSPHRDPAPRGPARFLPLPGL
PPALPEPPGILAPQPPDVGSSDPLSMVGPSQGRSPSYASRSVPGAGVPGAGVPGAGVPGAGVPGAGVPGA
GVPGAGVPGAGVPGVGVPGVGVPGVGVPGVGVPGVGVPGVGVPGVGVPGVGVPGVGVPGVGVPGVGVPGVG
VPGVGGSVPGAGVPGAGVPGAGVPGAGVPGAGVPGAGVPGAGVPGAGVPGVGVPGVGVPGVGVPGVGVPGV
GVPGVGVPGVGVPGVGVPGVGVPGVGVPGVGVPGVGVPGVG).
```

In the present disclosure, a fusion protein which comprises rhFGF21 having an amino acid sequence with at least 80% identity to SEQ ID NO: 1, or a fusion protein which comprises an amino acid sequence with at least 80% identity to SEQ ID NO: 2 has hypoglycemic activity.

In one embodiment, the present application provides a polynucleotide encoding the above-mentioned fusion protein. Unless stated otherwise, the terms "polynucleotide", "nucleic acid", "nucleic acid sequence" and "gene" herein are used interchangeably.

In some preferred embodiments, a portion of polynucleotide encoding rhFGF21 in the above-mentioned fusion protein comprises a nucleotide sequence set forth in SEQ ID NO: 3 or a nucleotide sequence with at least 80%, e.g., at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, 98%, or 99% identity to SEQ ID NO: 3.

In a further preferred embodiment, the portion of polynucleotide encoding rhFGF21 in the above-mentioned fusion protein has the nucleotide sequence set forth in SEQ ID NO: 3 (SEQ ID NO: 3:

```
CATCCTATTCCTGACAGCTCACCCCTGTTACAGTTTGGCGGTCAGGTTCGTCAACGCTATTTATACACC
GACGACGCACAGCAAACCGAAGCACACCTGGAAATTCGTGAAGACGGCACCGTTGGTGGTGCAGCA
GATCAATCCCCGGAATCTCTGCTGCAGTTAAAAGCATTAAAACCGGGCGTTATACAGATTTTAGGTGTC
AAAACCTCCCGTTTTCTGTGTCAGCGGCCGGACGGTGCGCTTTATGGTAGCTTACACTTTGATCCGGA
AGCCTGTAGTTTTCGTGAGCTGCTGCTGGAGGACGGTTACAATGTTTATCAATCAGAAGCACATGGTC
TGCCGTTACATCTGCCGGGTAATAAGAGCCCGCATCGCGATCCGGCACCGCGTGGTCCAGCCAGATTT
CTGCCTCTGCCGGGACTGCCGCCGGCACTTCCTGAACCGCCTGGTATTCTGGCACCGCAGCCGCCTGA
TGTTGGTTCATCTGATCCTCTGTCAATGGTTGGACCGAGCCAGGGACGTTCTCCCTCATACGCTTCT).
```

In some preferred embodiments, a portion of polynucleotide encoding the linker in the above-mentioned fusion protein comprises a nucleotide sequence set forth in SEQ ID NO: 4 and/or SEQ ID NO: 5, or a nucleotide sequence with at least 80%, e.g., at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, 98%, or 99% identity to SEQ ID NO: 4 and/or SEQ ID NO: 5.

In a further preferred embodiment, the portion of polynucleotide encoding the linker in the above-mentioned fusion protein has a nucleotide sequence set forth in SEQ ID NO: 4 and/or SEQ ID NO: 5 (SEQ ID NO: 4: AGATCT; SEQ ID NO: 5: GGATCC).

In some preferred embodiments, the polynucleotide encoding the above-mentioned fusion protein comprises a nucleotide sequence set forth in SEQ ID NO: 6 or a nucleotide sequence with at least 80%, e.g., at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, 98%, or 99% identity to SEQ ID NO: 6.

In a preferred embodiment, the polynucleotide encoding the above-mentioned fusion protein has a nucleotide sequence set forth in SEQ ID NO: 6 (SEQ ID NO: 6:

CATCCTATTCCTGACAGCTCACCCCTGTTACAGTTTGGCGGTCAGGTTCGTCAACGCTATTTATACACC
GACGACGCACAGCAAACCGAAGCACACCTGGAAATTCGTGAAGACGGCACCGTTGGTGGTGCAGCA
GATCAATCCCCGGAATCTCTGCTGCAGTTAAAAGCATTAAAAACCGGGCGTTATACAGATTTTAGGTGTC
AAAAACCTCCCGTTTTCTGTGTCAGCGGCCGGACGGTGCGCTTTATGGTAGCTTACACTTTGATCCGGA
AGCCTGTAGTTTTCGTGAGCTGCTGCTGGAGGACGGTTACAATGTTTATCAATCAGAAGCACATGGTC
TGCCGTTACATCTGCCGGGTAATAAGAGCCCGCATCGCGATCCGGCACCGCGTGGTCCAGCCAGATTT
CTGCCTCTGCCGGGACTGCCGCCGGCACTTCCTGAACCGCCTGGTATTCTGGCACCGCAGCCGCCTGA
TGTTGGTTCATCTGATCCTCTGTCAATGGTTGGACCGAGCCAGGGACGTTCTCCCTCATACGCTTCTAG
ATCTGTTCCGGGTGCAGGTGTTCCGGGTGCCGGTGTTCCGGGGGCAGGTGTTCCTGGTGCGGGTGTT
CCGGGCGCGGGTGTTCCTGGAGCGGGTGTTCCAGGTGCGGGTGTGCCGGGTGCGGGTGTACCTGGTG
TTGGTGTTCCGGGAGTTGGTGTTCCTGGGGTTGGTGTTCCAGGGGTTGGTGTGCCGGGGGTTGGTGT
CCCGGGTGTTGGTGTGCCTGGTGTTGGGGTTCCGGGTGTTGGGGTGCCGGGTGTTGGCGTTCCGGGT
GTGGGTGTTCCGGGTGTCGGTGTTCCGGGTGTAGGTGGATCTGTTCCGGGTGCAGGTGTTCCGGGTG
CCGGTGTTCCGGGGGCAGGTGTTCCTGGTGCGGGTGTTCCGGGCGCGGGTGTTCCTGGAGCGGGTGT
TCCAGGTGCGGGTGTGCCGGGTGCGGGTGTACCTGGTGTTGGTGTTCCGGGAGTTGGTGTTCCTGGG
GTTGGTGTTCCAGGGGTTGGTGTGCCGGGGGTTGGTGTCCCGGGTGTTGGTGTGCCTGGTGTTGGGG
TTCCGGGTGTTGGGGTGCCGGGTGTTGGCGTTCCGGGTGTGGGTGTTCCGGGTGTCGGTGTTCCGGG
TGTAGGTTGA）.

In one embodiment, the present application provides a recombinant expression vector containing a polynucleotide encoding the above-mentioned fusion protein.

Although there are many types of expression vectors at present, it is not easy to select a suitable vector for a specific protein, and while many expression systems have high protein expression amount, the expressed protein is of poor solubility. As for the fusion protein of the present application, in order to facilitate mass production and the subsequent purification, it is preferably able to be expressed in a prokaryotic expression system. Therefore, prokaryotic expression vectors suitable for a prokaryotic expression system are contemplated in the present application. Preferably, considering that a molecular chaperone will help to assist in protein folding, a prokaryotic expression vector carrying a molecular chaperone can be selected, wherein the molecular chaperone is preferably a small ubiquitin-like modifier protein (SUMO). SUMO is a small molecule ubiquitin-like modifier protein, which can facilitate the correct folding of the target protein and thus increases the expression and solubility of the target protein.

As a non-limiting example, appropriate expression vectors suitable for the present application include, for example, but are not limited to: pSUMO vector, PET series of vectors (e.g., PET30a vector).

In one embodiment, the present application provides a recombinant cell containing the above-mentioned recombinant expression vector or having the above-mentioned polynucleotide integrated in its genome.

Considering the convenience of the subsequent fermentation and purification, preferably, the host cell used to construct the recombinant cell is prokaryotic host cell. Preferably, examples of the prokaryotic host cell include, but are not limited to, *E. coli* (e.g., *E. coli Rossetta* (DE3)), *Agrobacterium tumefaciens, Staphylococcus aureus, Staphylococcus albus, Lactobacillus acidophilus, Bacillus anthracis, Bacillus subtilis,* or *Bacillus thuringiensis.,* etc.

In one embodiment, the present application provides a pharmaceutical composition, wherein the pharmaceutical composition comprises the above-mentioned fusion protein.

In a preferred embodiment, the pharmaceutical composition further comprises a pharmaceutically acceptable excipient. The pharmaceutically acceptable excipient can select from, for example, but is not limited to, solvents, propellants, solubilizers, co-solvents, emulsifiers, coloring agents, disintegrants, fillers, lubricants, wetting agents, osmotic pressure regulators, stabilizers, glidants, flavoring agents, preservatives, suspending agents, antioxidants, penetration enhancers, pH regulators, surfactants, diluents, and the like. Regarding other available pharmaceutically acceptable excipients, please refer to, for example, R.C. Rowe et al., translated mainly by Zemin Zheng, Handbook of Pharmaceutical Excipients, 4th ed., Chemical Industry Press, 2005.

In one embodiment, the present application relates to a kit, wherein the kit comprises the above-mentioned fusion protein or the above-mentioned pharmaceutical composition.

In some preferred embodiments, the kit further comprises a citrate buffer solution of pH 5.0-5.5.

In some preferred embodiments, the kit further comprises an aqueous arginine solution, preferably an aqueous arginine solution at a concentration of 50 mmol/L-150 mmol/L.

In some preferred embodiments, the kit further comprises an arginine + citrate buffer solution of pH 5.0-5.5.

In some preferred embodiments, the kit further comprises a citrate buffer solution of pH 5.0-5.5 and an aqueous arginine solution, and the fusion protein or the pharmaceutical composition, the citrate buffer solution of pH 5.0-5.5, and the aqueous arginine solution are packaged together or separately.

In one embodiment, the present application relates to use of the above-mentioned fusion protein in the preparation of a medicament for the prevention or treatment of a disease associated with abnormal glucose metabolism. In an alternative embodiment, the present application relates to a method of preventing or treating a disease associated with abnormal glucose metabolism, comprising administering to a subject in need thereof the above-mentioned fusion protein. In an alternative embodiment, the present application relates to a fusion protein for use in the prevention or treatment of a disease associated with abnormal glucose metabolism.

In a preferred embodiment, the disease associated with abnormal glucose metabolism is selected from, but not limited to, diabetes mellitus and diabetes-related metabolic disease. Preferably, the diabetes-related metabolic disease is selected from, but not limited to, diabetic nephropathy, dyslipidemia, obesity, cardiovascular diseases, metabolic syndrome, lipid metabolism disorders, non-alcoholic fatty liver diseases (NAFLD), or nervous system diseases (e.g., epilepsy, depression, etc.).

In one embodiment, the present application provides a method of producing the above-mentioned fusion protein of the present application, wherein the method comprises: culturing the recombinant cell, then inducing said recombinant cell to express and purifying the expression product to obtain the fusion protein.

In a preferred embodiment, the recombinant cell can be recombinant prokaryotic cell, preferably, examples of the recombinant prokaryotic cell include, but are not limited to, recombinant *E. coli* (e.g., recombinant *E. coli Rossetta* (DE3)), recombinant *Agrobacterium tumefaciens,* recombinant *Staphylococcus aureus,* recombinant *Staphylococcus albus,* recombinant *Lactobacillus acidophilus,* recombinant *Bacillus anthracis,* recombinant *Bacillus subtilis* or recombinant *Bacillus thuringiensis,* etc.

The culture of the recombinant cells can be carried out by those skilled in the art by selecting conventional culture medium and culture conditions according to the type of recombinant cells (please refer to, for example, the records in, Manufacture and Application of Microbial Medium, mainly edited by Tianshou Chen, China Agricultural Press, 1995; Practical Handbook of Microbial Medium, mainly edited by Lehao Ma et al., Jilin Science and Technology Press, 2005; http://www.cgmcc.net/; and https://www.sigmaaldrich.com/china-mainland/technical-documents/articles/biology/microbial-media.html, etc.).

The expression of the above-mentioned recombinant cells can be induced using conventional inducers known to those skilled in the art. As an exemplary embodiment, the expression of the above-mentioned recombinant cells is induced using, for example, an inducer selected from IPTG and/or lactose.

The expression product may be purified to obtain the fusion protein using conventional purification means known to those skilled in the art, the conventional purification means can be, for example, but are not limited to, ion exchange chromatography, adsorption chromatography, affinity chromatography, molecular sieve chromatography, hydrophobic chromatography, or any combination thereof. In a preferred embodiment, the purification of the expression product comprises: (i) after being induced to express, centrifuging the recombinant cell to collect bacteria, collecting a supernatant by centrifugation after breaking the bacteria; (ii) subjecting the supernatant to ion exchange chromatography and collecting an eluate; and (iii) subjecting the eluate to affinity chromatography to obtain the fusion protein.

In some preferred embodiments, the recombinant cells are centrifuged at 10,000-12,000 rpm/min to collect the bacteria.

In some preferred embodiments, the bacteria are broken by adding lysozyme to the bacteria and performing ultrasonication.

In some preferred embodiments, the supernatant is collected by performing the centrifugation at 10,000-12,000 rpm/min.

In some preferred embodiments, the ion exchange chromatography is performed using an agarose gel, such as DEAE agarose gel.

In some preferred embodiments, the supernatant is eluted using a buffer of pH 8.0-8.2 comprising 25 mmol/L Tris-HCl and 0.25 mol/L NaCl.

In some preferred embodiments, the affinity chromatography is performed using a Ni·NTA resin.

In some preferred embodiments, the affinity chromatography comprises: after equilibrating an affinity chromatography column with an equilibration buffer, loading the affinity chromatography column with the eluate and washing the same with a washing buffer, and then, eluting the target protein with an elution buffer.

In some preferred embodiments, the equilibration buffer comprises 50 mmol/L Tris-HCl, 0.5 mol/L NaCl, and 10 mmol/L imidazole, with pH 8.0-8.2.

In some preferred embodiments, the washing buffer comprises 50 mmol/L Tris-HCl, 500 mmol/L NaCl, and 50 mmol/L imidazole, with pH 8.0-8.2.

In some preferred embodiments, the elution buffer comprises 50 mmol/L Tris-HCl, 500 mmol/L NaCl, and 500 mmol/L imidazole, with pH 8.0-8.2.

In some preferred embodiments, the method of producing the above-mentioned fusion protein further comprises:

(1) synthesizing a polynucleotide encoding rhFGF21 and synthesizing a polynucleotide encoding (VPGXG)$_{20}$;
(2) performing enzyme digestion and ligation to the above-mentioned polynucleotide encoding rhFGF21 and the polynucleotide encoding (VPGXG)$_{20}$, and obtaining a polynucleotide encoding the fusion protein through PCR reaction;

(3) performing enzyme digestion and ligation to the polynucleotide encoding the fusion protein and an expression vector to obtain a recombinant expression vector, introducing the recombinant expression vector into a host cell, and screening to obtain the recombinant cell.

The polynucleotide sequence encoding rhFGF21 can be synthesized using conventional gene synthesis means through codon optimization on the basis of the protein sequence of rhFGF21 or the DNA or RNA sequence thereof which is known in the art (for example, please refer to https://www.ncbi.nlm.nih.gov/protein/NP_061986.1; https://www.ncbi.nim.nih.gov/nuccore/NG_033945.1; or https://www.ncbi.nlm.nih.gov/nuccore/NM_019113.4).

In a preferred embodiment, in step (1), the polynucleotide sequence encoding rhFGF21 comprises a nucleotide sequence set forth in SEQ ID NO: 3 or a nucleotide sequence with at least 80%, e.g., at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, 98%, or 99% identity to SEQ ID NO: 3.

In a preferred embodiment, in step (1), the polynucleotide encoding rhFGF21 has the nucleotide sequence set forth in SEQ ID NO: 3.

In a preferred embodiment, in step (1), the polynucleotide encoding $(VPGXG)_{20}$ comprises the nucleotide sequence set forth in SEQ ID NO: 7 or a nucleotide sequence with at least 80%, e.g., at least 81%, at least 82%, at least 83%, at least 84%, at least 85%, at least 86%, at least 87%, at least 88%, at least 89%, at least 90%, at least 91 %, at least 92%, at least 93%, at least 94%, at least 95%, at least 96%, at least 97%, 98%, or 99% identity to SEQ ID NO: 7.

In a preferred embodiment, in step (1), the polynucleotide encoding $(VPGXG)_{20}$ has a nucleotide sequence set forth in SEQ ID NO: 7 (SEQ ID NO: 7:

GTTCCGGGTGCAGGTGTTCCGGGTGCCGGTGTTCCGGGGGGCAGGTGTTCCTGGTGCGGGTGTTCCGG
GCGCGGGTGTTCCTGGAGCGGGTGTTCCAGGTGCGGGTGTGCCGGGTGCGGGTGTACCTGGTGTTGG
TGTTCCGGGAGTTGGTGTTCCTGGGGTTGGTGTTCCAGGGGTTGGTGTGCCGGGGGTTGGTGTCCCG
GGTGTTGGTGTGCCTGGTGTTGGGGTTCCGGGTGTTGGGGTGCCGGGTGTTGGCGTTCCGGGTGTGG
GTGTTCCGGGTGTCGGTGTTCCGGGTGTAGGTTGA).

In a preferred embodiment, in step (2), a polynucleotide encoding a linker selected from RS and/or GS is optionally attached to 5' end and 3' end of the polynucleotide encoding $(VPGXG)_{20}$, respectively. Preferably, polynucleotides having the nucleotide sequences set forth in SEQ ID NO: 4 and SEQ ID NO: 5 are attached to 5' end and 3' end of the polynucleotide encoding $(VPGXG)_{20}$, respectively.

In a preferred embodiment, in step (2), the enzyme digestion is performed using *Bgl*II restriction endonuclease and *Bam*HI restriction endonuclease.

Suitable conventional PCR experimental conditions and steps can be selected by those skilled in the art based on general knowledge in the art (please refer to, for example, Molecular Cloning: A Laboratory Manual (4th ed.), edited by J. Sambrook et al., translated mainly by Fuchu He, Science Press, 2017), or the PCR reaction can be performed using commercially available PCR kits according to the instructions therein. Without limitation, other primer pairs and other means of gene synthesis known in the art may also be used to obtain the polynucleotide encoding the above-mentioned fusion protein.

The above-mentioned enzyme digestion and ligation have the meaning well known in the art, wherein the enzyme digestion and ligation refers to a specific cleavage of two target DNA fragments or one target DNA fragment and a vector DNA molecule using a restriction nucleic acid endonuclease, followed by a ligation of the two target DNA fragments after enzyme digestion or the target DNA fragment and the ends of the vector DNA molecule after the enzyme digestion using a ligase, thereby obtaining the recombinant molecule or recombinant vector.

In a preferred embodiment, in step (3), *BsaI* restriction endonuclease and *Bam*HI restriction endonuclease are used to perform the enzyme digestion.

Regarding the particular means of introducing a recombinant expression vector into a host cell, any conventional means known in the art may be used, such as transformation, transfection, etc. Preferably, the recombinant expression vector is introduced into the host cell by transformation. Transformation refers to the processes of treating a host cell using some of known methods in molecular biology and genetic engineering so that the treated host cell is competent, and thereby contacting the host cell with exogenous DNA(s) so that the exogenous DNA(s) is introduced into the competent host cell.

In a preferred embodiment, in step (3), said host cell is a prokaryotic host cell. Preferably, examples of the prokaryotic host cell include, but are not limited to, *E. coli* (e.g., *E. coli Rossetta* (DE3)), *Agrobacterium tumefaciens, Staphylococcus aureus, Staphylococcus albus, Lactobacillus acidophilus, Bacillus anthracis, Bacillus subtilis,* or *Bacillus thuringiensis,* etc.

Regarding host cells into which a recombinant expression vector is introduced, positive recombinant cells can be obtained by screening using conventional means such as resistance screening. The culture of the recombinant cells can be carried out by those skilled in the art by selecting conventional culture medium and culture conditions according to the type of the recombinant cells (for example, please refer to the records in Manufacture and Application of Microbial Medium, mainly edited by Tianshou Chen, China Agricultural Press, 1995; Practical Handbook of Microbial Medium, mainly edited by Lehao Ma et al., Jilin Science and Technology Press, 2005; http://www.cgmcc.net/; and https://www.sigmaaldrich.com/china-mainland/technical-documents/articles/biology/microbial-media.html, etc.).

In a preferred embodiment, in step (3), the expression vector is selected from, but not limited to, pSUMO vector and PET series of vectors (e.g., PET30a vector). In a further preferred embodiment, pSUMO vector carrying a small molecule ubiquitin-like modifier protein (SUMO) as a molecular chaperone is used as the expression vector, and *E. coli Rossetta* (DE3) is used as the host cell.

In an exemplary embodiment, a recombinant pSUMO vector containing the polynucleotide encoding the fusion protein is transformed into the host cell; the recombinant cells obtained by screening are cultured, and the recombinant cells are induced to express the SUMO-fusion protein, and the expression product is purified by ion exchange chromatography and affinity chromatography to obtain a preliminarily purified SUMO-fusion protein, and after dialysis, the molecular chaperone SUMO linked to the fusion protein is cut using the SUMO protease, and the fusion protein after cutting of SUMO is purified by affinity chromatography to obtain the purified fusion protein.

Exemplary technical solutions of the present application can be illustrated by the following numbered paragraphs:

1. A recombinant human fibroblast growth factor 21 fusion protein, wherein the fusion protein comprises rhFGF21 and $(VPGXG)_n$ which are fused together, the rhFGF21 comprises an amino acid sequence set forth in SEQ ID NO: 1 or an amino acid sequence with at least 80% identity to SEQ ID NO: 1; X is A and/or V; and n is an integer selected from 20-80.

2. The fusion protein according to paragraph 1, wherein X is A and V, and the ratio of A to V is (1-3):(1-3), preferably 2:3.

3. The fusion protein according to paragraph 1 or 2, wherein n is an integer from 40 to 60, preferably 40.

4. The fusion protein according to any of paragraphs 1-3, wherein the fusion protein consists of rhFGF21, $(VPGXG)_n$, and an optional linker, which are fused together.

5. The fusion protein according to paragraph 4, wherein an amino acid sequence of the linker is RS and/or GS.

6. The fusion protein according to any of paragraphs 1-5, wherein the fusion protein has an amino acid sequence set forth in SEQ ID NO: 2 or an amino acid sequence with at least 80% identity to SEQ ID NO: 2.

7. A polynucleotide encoding the fusion protein according to any of paragraphs 1-6.

8. The polynucleotide according to paragraph 7, wherein a portion of polynucleotide encoding rhFGF21 in the fusion protein comprises a nucleotide sequence set forth in SEQ ID NO: 3 or a nucleotide sequence with at least 80% identity to SEQ ID NO: 3.

9. The polynucleotide according to paragraph 7 or 8, wherein a portion of polynucleotide encoding the linker in the fusion protein comprises a nucleotide sequence set forth in SEQ ID NO: 4 and/or SEQ ID NO: 5 or a nucleotide sequence with at least 80% identity to SEQ ID NO: 4 and/or SEQ ID NO: 5.

10. The polynucleotide according to any of paragraphs 7-9, wherein the polynucleotide comprises a nucleotide sequence shown by SEQ ID NO: 6, or a nucleotide sequence with at least 80% identity to SEQ ID NO: 6.

11. A recombinant expression vector comprising the polynucleotide according to any of paragraphs 7-10.

12. The recombinant expression vector according to paragraph 11, wherein the recombinant expression vector is a prokaryotic recombinant expression vector.

13. The recombinant expression vector according to paragraph 12, wherein the recombinant expression vector is a prokaryotic recombinant expression vector carrying a molecular chaperone.

14. The recombinant expression vector according to paragraph 13, wherein the molecular chaperone is a small molecule ubiquitin-like modified protein.

15. The recombinant expression vector according to paragraph 11 or 12, wherein the recombinant expression vector is a recombinant pSUMO vector or recombinant PET series of vectors.

16. A recombinant cell comprising the recombinant expression vector according to any of paragraphs 11-15 or having a polynucleotide according to any of paragraphs 7-10 integrated in its genome.

17. The recombinant cell according to paragraph 16, wherein a host cell for constructing the recombinant cell is a prokaryotic host cell.

18. The recombinant cell according to paragraph 17, wherein the prokaryotic host cell is selected from *E. coli, Agrobacterium tumefaciens, Staphylococcus aureus, Staphylococcus albus, Lactobacillus acidophilus, Bacillus anthracis, Bacillus subtilis,* or *Bacillus thuringiensis.*

19. A pharmaceutical composition comprising the fusion protein according to any of paragraphs 1-6.

20. The pharmaceutical composition according to paragraph 19, wherein the pharmaceutical composition further comprises a pharmaceutically acceptable excipient.

21. A kit comprising the fusion protein according to any of paragraphs 1-6 or the pharmaceutical composition according to paragraph 19 or 20.

22. The kit according to paragraph 21, wherein the kit further comprises a citrate buffer solution of pH 5.0-5.5.

23. The kit according to paragraphs 21 or 22, wherein the kit further comprises an aqueous arginine solution, preferably an aqueous arginine solution at a concentration of 50 mmol/L-150 mmol/L.

24. The kit according to any of paragraphs 21-23, wherein the kit further comprises an arginine + citrate buffer solution of pH 5.0-5.5.

25. The kit according to paragraph 21, wherein the kit further comprises an aqueous arginine solution and a citrate buffer solution of pH 5.0-5.5, and the fusion protein or the pharmaceutical composition, the citrate buffer solution of pH 5.0-5.5, and the aqueous arginine solution are packaged together or separately.

26. Use of the fusion protein according to any of paragraphs 1-6 in the preparation of a medicament for the prevention or treatment of a disease associated with abnormal glucose metabolism.

27. The use according to paragraph 26, wherein the disease associated with abnormal glucose metabolism is selected from diabetes mellitus and diabetes-related metabolic disease, preferably, the diabetes-related metabolic disease is selected from diabetic nephropathy, dyslipidemia, obesity, cardiovascular diseases, metabolic syndrome, lipid metabolism disorders, non-alcoholic fatty liver diseases, or nervous system diseases.

28. A method of producing the fusion protein according to any of paragraphs 1-6, comprising: culturing the recombinant cell according to any of paragraphs 16-18, then inducing the recombinant cell to express and purifying an expression product to obtain the fusion protein.

29. The method according to paragraph 28, wherein the expression of the recombinant cell is induced using an inducer selected from IPTG and/or lactose.

30. The method according to paragraph 28 or 29, wherein the purification of the expression product comprises: (i) after being induced to express, centrifuging the recombinant cell to collect bacteria, collecting a supernatant by centrifugation after breaking the bacteria; (ii) subjecting the supernatant to ion exchange chromatography and collecting an eluate; and (iii) subjecting the eluate to affinity chromatography to obtain the fusion protein.

31. The method according to any of paragraphs 28-30, further comprising:

(1) synthesizing a polynucleotide encoding rhFGF21 and synthesizing a polynucleotide encoding $(VPGXG)_{20}$;
(2) performing enzyme digestion and ligation to the polynucleotide encoding rhFGF21 and the polynucleotide encoding $(VPGXG)_{20}$, and obtaining a polynucleotide encoding the fusion protein through PCR reaction;

(3) performing enzyme digestion and ligation to the polynucleotide encoding the fusion protein and an expression vector to obtain a recombinant expression vector, introducing the recombinant expression vector into a host cell, and screening to obtain the recombinant cell.

32. The method according to paragraph 31, in step (1), the polynucleotide sequence encoding rhFGF21 comprises a nucleotide sequence set forth in SEQ ID NO: 3 or a nucleotide sequence with at least 80% identity to SEQ ID NO: 3.

33. The method according to paragraph 31 or 32, in step (1), the polynucleotide encoding $(VPGXG)_{20}$ comprises a nucleotide sequence set forth in SEQ ID NO: 7 or a nucleotide sequence with at least 80% identity to SEQ ID NO: 7.

34. The method according to any of paragraphs 31-33, in step (2), a polynucleotide encoding a linker selected from RS and/or GS is optionally attached to 5' end and 3' end of the polynucleotide encoding $(VPGXG)_{20}$, respectively.

35. The method according to any of paragraphs 31-34, in step (2), the enzyme digestion is performed using *Bg*/II restriction endonuclease and *Bam*HI restriction endonuclease.

36. The method according to any of paragraphs 31-35, in step (3), the enzyme digestion is performed using *BsaI* restriction endonuclease and *Bam*HI restriction endonuclease.

37. The method according to any of paragraphs 31-36, in step (3), the expression vector is selected from pSUMO vector and PET series of vectors.

38. The method according to any of paragraphs 31-37, in step (3), the expression vector is pSUMO vector and the host cell is *E. coli Rossetta* (DE3).

It should be noted that the operation steps involved in this application, such as the design, synthesis and cloning of genes, construction of prokaryotic expression vectors, nucleic acid extraction and sequence analysis and identification, as well as isolation and purification of expression products, can be performed according to techniques known in the art (for example, please refer to the records in *CURRENT PROTOCOLS IN MOLECULAR BIOLOGY*). If not specifically indicated, the technical means used in the examples are conventional means known to those skilled in the art. Unless stated otherwise, the reagents, materials, and equipment used in the following examples are commercially available.

**EXAMPLES**

**Example 1** Preparation of the gene of recombinant human fibroblast growth factor 21 fusion protein
The gene of rhFGF21 and the gene of $(VPGXG)_{20}$ were ligated by the *Bg*/II restriction site, and then PCR was performed to introduce *BsaI* and *Bam*HI restriction sites at 5' end and 3' end of rhFGF21-$(VPGXG)_{20}$; then rhFGF21-$(VPGXG)_{20}$ was ligated to another gene of $(VPGXG)_{20}$ which had undergone *Bg*/II and *Bam*HI enzyme digestions using the *Bam*HI restriction site to obtain rhFGF21-$(VPGXG)_{40}$. Then PCR was performed to introduce the *BsaI* restriction site at 5' end of rhFGF21-$(VPGXG)_{40}$ and to introduce the stop codon and *Bam*HI restriction site at 3' end of rhFGF21-$(VPGXG)_{40}$.

1. Synthesis of the gene of rhFGF21 carrying enzyme restriction sites

The amino acid sequence of wild-type hFGF21 (SEQ ID NO: 1) was searched on NCBI, and the nucleotide sequence (SEQ ID NO: 3) encoding rhFGF21 was obtained via codon optimization. Synthesis of the gene (SEQ ID NO: 8) carrying the *BsaI* enzyme restriction site (SEQ ID NO: 10: GGTCTCAGGT) and the *Bg*/II enzyme restriction site (SEQ ID NO: 4: AGATCT) was entrusted to a gene synthesis company (SEQ ID NO: 8:

```
GGTCTCAGGTCATCCTATTCCTGACAGCTCACCCCTGTTACAGTTTGGCGGTCAGGTTCGTCAACGCTA
TTTATACACCGACGACGCACAGCAAACCGAAGCACACCTGGAAATTCGTGAAGACGGCACCGTTGGT
GGTGCAGCAGATCAATCCCCGGAATCTCTGCTGCAGTTAAAAGCATTAAAACCGGGCGTTATACAGAT
TTTAGGTGTCAAAAACCTCCCGTTTTCTGTGTCAGCGGCCGGACGGTGCGCTTTATGGTAGCTTACACT
TTGATCCGGAAGCCTGTAGTTTTCGTGAGCTGCTGCTGGAGGACGGTTACAATGTTTATCAATCAGAA
GCACATGGTCTGCCGTTACATCTGCCGGGTAATAAGAGCCCGCATCGCGATCCGGCACCGCGTGGTCC
AGCCAGATTTCTGCCTCTGCCGGGACTGCCGCCGGCACTTCCTGAACCGCCTGGTATTCTGGCACCGC
AGCCGCCTGATGTTGGTTCATCTGATCCTCTGTCAATGGTTGGACCGAGCCAGGGACGTTCTCCCTCAT
ACGCTTCTAGATCT〉.
```

2. Preparation of the gene of linker-(VPGXG)$_{20}$

Synthesis of the gene of linker-(VPGXG)$_{20}$ having the nucleotide sequence set forth in SEQ ID NO: 9 was entrusted to a gene synthesis company, wherein the gene was consisted of a polynucleotide encoding the linker RS having the nucleotide sequence set forth in SEQ ID NO: 4 (i.e., the *Bg*/II restriction site) at 5' end, a polynucleotide encoding (VPGXG)$_{20}$ having the nucleotide sequence set forth in SEQ ID NO: 7, and a polynucleotide encoding the linker GS having the nucleotide sequence set forth in SEQ ID NO: 5 at 3' end (i.e., the *Bam*HI restriction site) (SEQ ID NO: 9:

AGATCTGTTCCGGGTGCAGGTGTTCCGGGTGCCGGTGTTCCGGGGGCAGGTGTTCCTGGTGCGGGTG
TTCCGGGCGCGGGTGTTCCTGGAGCGGGTGTTCCAGGTGCGGGTGTGCCGGGTGCGGGTGTACCTGG
TGTTGGTGTTCCGGGAGTTGGTGTTCCTGGGGTTGGTGTTCCAGGGGTTGGTGTGCCGGGGGTTGGT
GTCCCGGGTGTTGGTGTGCCTGGTGTTGGGGTTCCGGGTGTTGGGGTGCCGGGTGTTGGCGTTCCGG
GTGTGGGTGTTCCGGGTGTCGGTGTTCCGGGTGTAGGTGGATCC）.

3. Preparation of the gene of rhFGF21-(VPGXG)$_{40}$ fusion protein

The gene of rhFGF21 carrying the restriction site and the gene of linker-(VPGXG)$_{20}$ obtained above were used as templates. Enzyme digestion was performed using *Bg*/II restriction endonuclease according to the instructions of the manufacturer and ligation was performed using T4 DNA ligase to obtain the nucleotide sequence of rhFGF21-(VPGXG)$_{20}$ polypeptide.

In order to obtain the gene of the rhFGF21-(VPGXG)$_{40}$ fusion protein, PCR was performed using primers P1 (SEQ ID NO: 11) and P2 (SEQ ID NO: 12) and rTaq enzyme according to the 50 μL system described below, and the *Bsa*I and *Bam*HI restriction sites were introduced to 5' end and 3' end of the nucleotide sequence of the obtained rhFGF21-(VPGXG)$_{20}$ polypeptide.

    P1: 5'-GGTCTCAGGTCATCCTATTCCTGACAGCT-3' (SEQ ID NO: 11)
    *Bsa*I
    P2: 5'-GGATCCACCTACACCCGGAACA-3' (SEQ ID NO: 12)
    *Bam*HI

After the above-mentioned nucleotide sequence introduced with the restriction sites was ligated to T vector using solution I ligase, it was digested with *Bam*HI restriction endonuclease; meanwhile, the gene of linker-(VPGXG)$_{20}$ was digested with *Bg*/II and *Bam*HI restriction endonucleases according to the instructions of the manufacturer, and the above two enzyme-digested products were extracted from gel using the DNA fragment gel extraction kit (purchased from Omega, Cat. No.: D6492) and then ligated with T4 DNA ligase.

Then, the PCR reaction (50 uL system) was performed using rTaq enzyme with the ligated product as a template, and the stop codon (TCA) was introduced:

| | |
|---|---|
| 10×PCR buffer | 5.0 μL |
| dNTPs | 4.0 μL |
| template | 2.0 μL (10 ng) |
| P1 | 1.0 μL (10 pmol) |
| P3 | 1.0 μL (10 pmol) |
| rTaq enzyme | 0.5 μL |
| ddH$_2$O | 36.5 μL |

wherein the sequences of primer P1 and primer P3 (SEQ ID NO: 13) were as follows.

    P1: 5'-GGTCTCAGGTCATCCTATTCCTGACAGCT-3' (SEQ ID NO: 11)
    *Bsa*I
    P3: 5'-GGATCCTCAACCTACACCCGGAACA-3' (SEQ ID NO: 13)
    *Bam*HI

The obtained PCR products were extracted from gel using the DNA fragment gel extraction kit (purchased from Omega, Cat. No.: D6492) to obtain the gene of the rhFGF21-(VPGXG)$_{40}$ fusion protein.

4. Preparation of genes of other fusion proteins

The genes of the rhFGF21-(VPGXG)$_{20}$, rhFGF21-(VPGXG)$_{60}$, and rhFGF21-(VPGXG)$_{8O}$ fusion proteins were obtained according to the same operations and conditions as those described above for the preparation of the gene of the rhFGF21-(VPGXG)$_{40}$ fusion protein.

Wherein, in order to prepare the gene of the rhFGF21-(VPGXG)$_{20}$ fusion protein, the stop codon was introduced directly at 3' end of the nucleotide sequence of the obtained rhFGF21-(VPGXG)$_{20}$ polypeptide via PCR, without additionally introducing restriction sites, and in which the 50 $\mu$L PCR system was the same as described above.

**Example 2** Preparation of Human Fibroblast Growth Factor 21 Fusion Protein

The pSUMO and *E. coli Rossetta* (DE3) involved in this example were commercially available

1. Construction of recombinant prokaryotic expression vector for the rhFGF21-(VPGXG)$_{40}$ gene

The fragment of the target gene rhFGF21-(VPGXG)$_{40}$ obtained in Example 1 and the prokaryotic expression vector pSUMO were double digested with *Bsal* and *Bam*HI enzymes according to the instructions of the manufacturer, and the digested product was purified using the DNA fragment gel extraction kit (purchased from Omega, Cat. No.: D6492), followed by the ligation using T4 DNA ligase in a 10 $\mu$L ligation reaction system according to the instructions of the manufacturer overnight at 16°C.

After double digestion with *Bsal* and *Bam*HI enzymes and identification of the digested product by agarose gel electrophoresis, the recombinant plasmid pSUMO-rhFGF21-(VPGXG)$_{40}$ containing the correct sequence was constructed.

2. Expression and purification of the rhFGF21-(VPGXG)$_{40}$ protein

(1) Induced expression

The recombinant plasmid pSUMO-rhFGF21-(VPGXG)$_{40}$ containing the correct sequence was transformed into the expression strain, *E. coli Rossetta* (DE3) competent cells. The transformed competent cells were plated onto LB plates containing 100 $\mu$g/mL ampicillin, and incubated at 37°C for 10-12 h before selecting the single colony of the transformed positive recombinant bacteria and inoculating it into 5 mL LB medium containing ampicillin (100 ug/mL) and then incubating the same at 37°C for 10 h. The culture solution was inoculated into 500 mL LB medium containing ampicillin (100 pg/mL) in the proportion of 1:100, and was cultured at 37°C for 2 h. When A600=0.3-0.6, IPTG was added to a final concentration of 0.5 mmol/L for induction (prior to the induction, the culture was taken and centrifuged at 12000 rpm/min for 30 min to obtain supernatant and precipitate, and the supernatant and precipitate were analyzed by 15% SDS-PAGE electrophoresis). After being induced for 3h, the bacteria were collected under centrifugation for 30 min at 12000 rpm/min.

(2) Protein purification

After collecting the bacteria through the above-described centrifugation, lysozyme was added into the bacteria to a final concentration of 1 mg/mL, which was left on ice for 1 h. The bacteria were then broken with ultrasound and centrifugated (12,000 rpm/min, 30 min) after the completion of the breaking to collect the supernatant. The obtained supernatant and precipitate were taken and analyzed by 15% SDS-PAGE electrophoresis, in which the soluble expressed target protein can be 70% or more of the total target protein in the bacteria. The supernatant was purified through DEAE Sepharose FF chromatography column, wherein the elution was performed with buffer 1 (25 mmol/L Tris-HCl, 0.25 mol/L NaCl, pH 8.0) and the ion exchange chromatography eluate was collected. The ion exchange chromatography eluate was subjected to Ni·NTA resin column affinity chromatography, in which the Ni·NTA resin column was firstly equilibrated with equilibration buffer (50 mmol/L Tris-HCl, pH 8.0, 0.5 mol/L NaCl, 10 mmol/L imidazole), and then the above-mentioned eluate was loaded into the equilibrated resin column. After finishing the loading, washing buffer (50 mmol/L Tris-HCl, pH 8.0, 0.5 mol/L NaCl, 50 mmol/L imidazole) was used for washing. Finally, the target protein was eluted with elution buffer (50 mmol/L Tris-HCl, pH 8.0, 0.5 mol/L NaCl, 500 mmol/L imidazole). The solution of each elution peak was collected to obtain the affinity chromatography eluate, which was subjected to SDS-PAGE assay.

The affinity chromatography eluate was dialyzed with PBS buffer (pH 7.4) at 4°C overnight to obtain the fusion protein SUMO-rhFGF21-(VPGXG)$_{40}$ with imidazole removed, into which DTT was added to a final concentration of 2 mmol/L and SUMO Protease-I (with His Tag) (the cleavage ratio was 1:50, i.e., 1 mg SUMO Protease-I: 50 mg SUMO-rhFGF21-(VPGXG)$_{40}$), and the cleavage was carried out overnight at 4°C. The obtained cleavage product was mixed thoroughly with Ni-NTA Agarose to collect the flow-through liquid, wherein the non-specifically adsorbed rhFGF21-(VPGXG)$_{40}$ protein was eluted with PBS buffer (pH 7.4). After HPLC analysis (the mobile phase was PBS buffer at a flow rate of 1 mL/min, column: Agilent AdvanceBio SEC 300 Å, 2.7 $\mu$m, 7.8 × 300 mm, Cat. No.: PL1180-5301), it was shown that the purified rhFGF21-(VPGXG)$_{40}$ protein (having the amino acid sequence set forth in SEQ ID NO: 2) had a purity of 95% or more (Figure 3).

The expression and purification of rhFGF21-(VPGXG)$_{20}$, rhFGF21-(VPGXG)$_{60}$, and rhFGF21-(VPGXG)$_{80}$ proteins were performed with the same expression and purification operations and conditions as described above for the rhFGF21-(VPGXG)$_{40}$ protein. The expression amount of each protein was analyzed using 15% SDS-PAGE electrophoresis (Figure 1).

**Example 3 Activity test of human fibroblast growth factor 21 fusion protein**

1. Activity assay of the rhFGF21-(VPGXG)$_{40}$ protein at cellular level

β-Klotho was cloned into the retroviral eukaryotic expression vector pBMN-β Klotho-IRES-EGFP, and the green fluorescent protein was used as a reporter gene. The recombinant retroviral vector was transfected into 293 packaging cells with liposome transfection, and the viral supernatant was collected by centrifugation, which was used to infect 3T3-L1 precursor cells (purchased from ATCC, Cat No.: CL-173). β-Klotho-3T3-L1 stable cell line expressing β-Klotho, known as β-Klotho-3T3-L1 adipocyte, was screened using flow cytometry. The β-Klotho-3T3-L1 adipocytes were starved for 12 h. The purified rhFGF21-(VPGXG)$_{40}$ protein was diluted with cell culture medium (DMEM, gibco, C11965500BT) to the final concentrations of 10 nmol/L, 100 nmol/L, and 1000 nmol/L, and dilutions of the different concentrations were added in an amount of 1 ml per well into the differentiated mature adipocytes. At least 3 replicate wells were set for each concentration.

Detection of glucose concentration: after the cells treated above were incubated for 24 h, 2 $\mu$L of the supernatant of the medium was taken into 200 $\mu$L of glucose assay solution to measure the content of glucose. Each concentration was at least triplicate, and after reaction for 5-10 min at 37°C, the OD value was measured at 500 nm wavelength. The glucose consumption ratio of cells was calculated as follows, and the experimental results were statistically analyzed.

The residual glucose concentration (C) in the culture medium was calculated with the formula:

$$\text{Glucose concentration (mmol/L)} = OD_{sample}/OD_{standard} \times 5.55 \text{ mmol/L}$$

The glucose consumption ratio of cells was calculated with the formula:

$$\text{Glucose consumption ratio of cells (\%)} = [(C_{glucose\ of\ mock\ group} - C_{glucose\ of\ administration\ group})/ C_{glucose\ of\ mock\ group}] \times 100\%.$$

After treating the adipocytes with different concentrations of rhFGF21-(VPGXG)$_{40}$ protein for 24 h, the content of glucose in the culture medium was measured by the microscale glucose assay kit (GOD-POD method), and the result of statistical analysis showed that the uptake and utilization of glucose by the cells treated with rhFGF21-(VPGXG)$_{40}$ protein had significantly increased, and the content of glucose remaining in the culture medium significantly decreased, compared to the control group which received no treatment.

β-Klotho-3T3-L1 adipocytes were treated with rhFGF21 and each of the above-prepared fusion proteins following the above method, and the results showed that the glucose uptake of the untreated β-Klotho-3T3-L1 adipocytes was about 27%, which increased to about 35% after being stimulated with 10 nmol/L rhFGF21 , increased to about 39% after being stimulated with 10 nmol/L rhFGF21-(VPGXG)$_{20}$, increased to about 46% after being stimulated with 10 nmol/L rhFGF21-(VPGXG)$_{40}$, increased to about 37% after being stimulated with 10 nmol/L rhFGF21-(VPGXG)$_{60}$, and increased to about 32% after being stimulated with 10 nmol/L rhFGF21-(VPGXG)$_{80}$. The results showed that 10 nmol/L rhFGF21-(VPGXG)$_{40}$ fusion protein had the best activity compared to the same dose of rhFGF21 and other fusion proteins (Figure 2).

2. Comparison of the in vivo hypoglycemic activity of rhFGF21 and different fusion proteins in animals

Diabetic mice were obtained by inducing C57BL/6J mice (Changchun Yisi Experimental Animal Technology Co., Ltd., Animal Quality Certificate No.: SCXK (Ji)-2011-0004) with STZ (80 mg/kg), and the mice with a blood glucose consistently higher than 13.8 mmol/L were considered as diabetic mice.

The established diabetic model mice were randomly divided into 6 groups with 8 mice/group. Meanwhile, a normal mice group with 8 mice was used as the mock control group. By subcutaneous administration, mice in the model control group were injected with PBS buffer, the mice in the rhFGF21-(VPGXG)$_{40}$ treatment group were injected with the fusion protein at a dose of 1 mg/kg/d, and the mice in rhFGF21-(VPGXG)$_{20}$, rhFGF21-(VPGXG)$_{60}$ and rhFGF21-(VPGXG)$_{80}$ treatment groups were injected with the same dose of fusion protein as rhFGF21-(VPGXG)$_{40}$ treatment group. 12 hours after the injection was completed (during which the mice were fed *ad libitum*), the blood glucose levels of mice in each group were measured. The results showed that after 12 hours of administration, the hypoglycemic effect of the rhFGF21 treatment group decreased compared to the model group; each fusion protein treatment group showed certain therapeutic effects, among others, the blood glucose level of the mice in the rhFGF21-(VPGXG)$_{40}$ fusion protein treatment group significantly decreased compared to the model group (p<0.01), and the rhFGF21-(VPGXG)$_{40}$ treatment group had the best hypoglycemic effect compared to the other groups (Figure 4).

3. In vivo activity test of the rhFGF21-(VPGXG)$_{40}$ protein

(1) Detection of rhFGF21-(VPGXG)$_{40}$ protein activity with diabetic mice model

Diabetic mice (blood glucose is consistently higher than 13.8 mmol/L) were obtained by inducing C57BL/6J mice (Changchun Yisi Experimental Animal Technology Co., Ltd., Animal Quality Certificate No.: SCXK (Ji)-2011-0004) with STZ (80 mg/kg), and were randomly divided into 5 groups with 8 mice/group. Meanwhile, 8 normal mice were used as normal control group. By subcutaneous administration, the mice in the model control group were injected with PBS buffer; the mice in rhFGF21-(VPGXG)$_{40}$ treatment group were injected with the fusion protein at a dose of 1 mg/kg/d for continuous 58 days. The experiment started at 8:00 a.m. (the blood glucose of the model animals at this time was at a relatively high value within the day) and the mice were fed *ad libitum* during the experiment.

Model control group: diabetic mice were subcutaneously injected with PBS buffer at around 8:00 a.m., and the single injection volume is 0.15 mL.

rhFGF21-(VPGXG)$_{40}$ treatment group: diabetic mice were subcutaneously injected with the rhFGF21-(VPGXG)$_{40}$ fusion protein (the solvent was PBS buffer) at around 8:00 a.m. per day, the single injection dose is 1 mg/kg/d rhFGF21-(VPGXG)$_{40}$ fusion protein (in the amount of total protein), and the single injection volume is 0.15 mL.

Normal control group: healthy mice were subcutaneously injected with PBS buffer at around 8:00 a.m. per day, and the single injection volume is 0.15 mL.

The blood glucose of each group of mice was measured every 2 days, and the results showed that the blood glucose level of mice in the group treated with rhFGF21-(VPGXG)$_{40}$ fusion protein for 3 days significantly decreased compared to that of the model group, and the blood glucose thereof was maintained at a relatively low level and was close to the blood glucose level of mice in the normal group after 6 days of treatment. After continuous 30 days of administration, the glucose tolerance level of mice in each group was measured by OGTT. The results showed that the blood glucose of mice in the model group increased significantly within 30 min after the oral administration of glucose, and decreased slowly after 30 min. The blood glucose of mice in the rhFGF21-(VPGXG)$_{40}$ treatment group was significantly lower than that of the model group, and the blood glucose had decreased to be close to that of mice in the normal group at 60 min (Figure 5). The results showed that the glucose tolerance of mice was significantly improved after being treated with rhFGF21-(VPGXG)$_{40}$ fusion protein. During the 58-day injection, the blood glucose of mice in the model group was always maintained at a relatively high level, and the blood glucose of mice in the rhFGF21-(VPGXG)$_{40}$ treatment group significantly decreased compared to that of the model group, the blood glucose was maintained at a relatively low level and was close to that of mice in the normal group after the treatment, and the efficacy lasted longer than that of rhFGF21 (Figure 6). The serum insulin level of mice in the model group significantly decreased compared to that of the mice in normal group, and after being treated with the rhFGF21-(VPGXG)$_{40}$ fusion protein, the serum insulin content significantly increased compared to the model group (Figure 7). The glycated hemoglobin level of mice in the model group significantly increased compared to the normal group, and compared to the model group, the glycated hemoglobin significantly decreased after being treated with the rhFGF21-(VPGXG)$_{40}$ fusion protein (Figure 8). The G6Pase and PCK expression amounts in liver were measured by real-time fluorescence quantitative PCR and western blot. The results showed that the G6Pase and PCK expression amounts of the mice in the model group significantly increased compared to the normal group; and after being treated with rhFGF21-(VPGXG)$_{40}$, the G6Pase and PCK expression amounts in mice significantly decreased compared to the model group (Figure 9 to Figure 12). In addition, the damage of islet was evaluated using HE staining and immunohistochemistry. The results showed that the islets of model group mice were damaged severely, with the amount of the secreted insulin being significantly decreased; while the islet damage in the mice of the rhFGF21-(VPGXG)$_{40}$ treatment group was significantly improved and the amount of the secreted insulin significantly increased (Figure 13).

**Example 4** Determination of conditions for stably storing the human fibroblast growth factor 21 fusion protein

1. Determination of buffer solution for the rhFGF21-(VPGXG)$_{40}$ protein

The following buffers were prepared: acetate buffer solutions of pH 4.0 and pH 5.0 (prepared by dissolving acetic acid in water); citrate buffer solutions of pH 5.0, pH 5.5, and pH 6.0 (prepared by dissolving citric acid and sodium citrate in water); phosphate buffer solutions of pH 6.5 and pH 7.0 (prepared by dissolving disodium hydrogen phosphate and sodium dihydrogen phosphate in water); Tris-HCl buffer solutions of pH 7.5 and pH 8.0 (prepared by dissolving Tris-HCl in water). HiPrepTM 26/10 Desalting column was connected to the AKTA purifier 100 system, and firstly, the protective solution (20% ethanol) in the desalting column was flushed out with 8-10 times the column volume of distilled water, then the desalting column was equilibrated with 8-10 times the column volume of the above target buffer solution, and the sample (a solution of rhFGF21-(VPGXG)$_{40}$ protein with a concentration of 2 mg/mL in phosphate buffer of pH 7.4) was loaded. The UV curve raised first. When the UV curve dropped to the baseline, the conductance curve raised, and the protein at the corresponding UV absorption peak was collected using a collector, i.e., the rhFGF21-(VPGXG)$_{40}$ proteins replaced into buffer solutions were obtained. The nine groups of rhFGF21-(VPGXG)$_{40}$ proteins replaced into buffer solutions were stored in 25°C incubator away from light, and the appearances of the preserved proteins were checked at regular intervals, and they were sampled for 15% SDS-PAGE electrophoresis and HPLC assay (PBS buffer, 1 mL/min, chromatographic column: Agilent AdvanceBio SEC 300 Å, 2.7 $\mu$m, 7.8x300 mm, Cat. No.: PL1180-5301); and aqueous solution of rhFGF21-(VPGXG)$_{40}$ protein stored at -80°C was used as control.

The results showed that the rhFGF21-(VPGXG)$_{40}$ protein appeared turbid to the naked eye immediately after it was replaced into the acetate buffer solution of pH 4.0; and it appeared turbid on the 7$^{th}$ day in the acetate buffer solution of pH 4.5, appeared turbid on the 3$^{rd}$ day in the Tris-HCl buffer solution of pH 8.0, and appeared turbid on the 7$^{th}$ day in the Tris-HCl buffer solution of pH 7.5; and it did not appear turbid to the naked eye at the 14$^{th}$ day in all of the citrate buffer solutions and phosphate buffer solutions with any pH value. The proteins preserved in different buffers were sampled for 15% SDS-PAGE analysis and HPLC assay. From the results of 15% SDS-PAGE, it can be seen that although there was no turbidity visible to the naked eye in the rhFGF21-(VPGXG)$_{40}$ protein solutions preserved in phosphate buffer solutions of the two pH values, degradation of most of the protein occurred in both; and degradation of most of the protein also occurred in the rhFGF21-(VPGXG)$_{40}$ protein preserved in citrate buffer solution of pH 6.0. In contrast, the rhFGF21-(VPGXG)$_{40}$ protein stored in citrate buffer solutions of pH 5.0 and 5.5 showed relatively less degradation. It can be seen from the HPLC results that only the rhFGF21-(VPGXG)$_{40}$ protein preserved in citrate buffer solutions of pH 5.0 and pH 5.5 maintained relatively good integrity, using the aqueous solution of rhFGF21-(VPGXG)$_{40}$ protein without protectant stored at -80°C as control (Figure 14). And it was found from the results that the citrate buffer solution of pH 5.5 had a slightly better protection to the rhFGF21-(VPGXG)$_{40}$ protein than the citrate buffer solution of pH 5.0.

2. Determination of protectants for the rhFGF21-(VPGXG)$_{40}$ protein

The following solutions of protectants were prepared with distilled water: sucrose solutions (25 mg/mL, 50 mg/mL, 100 mg/mL), trehalose solutions (25 mg/mL, 50 mg/mL, 100 mg/mL), glycine solutions (50 mmol/L, 100 mmol/L, 150 mmol/L), arginine solutions (50 mmol/L, 100 mmol/L, 150 mmol/L), histidine solutions (25 mmol/L, 50 mmol/L, 100 mmol/L), mannitol solutions (2.5%, 5%, 7.5%), sorbitol solutions (5%, 10%, 15%), NaCl solutions (50 mg/mL, 100 mg/mL, 150 mg/mL), and dextran solutions (2%, 3%, 5%). The method of replacement for each solution of protectants was as described in Section 1 of this Example, and the protein at the corresponding UV absorption peak was collected using a collector, i.e., the rhFGF21-(VPGXG)$_{40}$ proteins replaced into protectants were obtained. The rhFGF21-(VPGXG)$_{40}$ proteins replaced into protectants in each group were stored in 25 °C incubator away from light, and sampled at regular intervals for 15% SDS-PAGE electrophoresis and HPLC assay (PBS buffer, 1 mL/min, chromatographic column: Agilent AdvanceBio SEC 300 Å, 2.7 $\mu$m. 7.8x300 mm, Cat No.: PL1180-5301), and aqueous solution of the rhFGF21-(VPGXG)$_{40}$ protein without protectant stored at -80°C was used as control.

As shown by the results of the 15% SDS-PAGE protein electrophoregram, the proteins preserved in solutions of other protectants had basically been completely degraded after being stored for 25 days at 25°C; while the rhFGF21-(VPGXG)$_{40}$ protein in arginine solution degraded relatively less and still had good integrity after being stored for 25 days at 25°C. It can be seen that the aqueous arginine solution had a very good protection effect on the stability of the fusion protein, and the differences among the protection effects of the aqueous arginine solutions of three different concentrations were not significant. For better comparison, the fusion proteins preserved in the aqueous arginine solutions of the above three concentrations were analyzed by HPLC (PBS buffer, 1 mL/min, chromatographic column: Agilent AdvanceBio SEC 300 Å 2.7 um, 7.8x300 mm, Cat. No.: PL1180-5301). From the results of HPLC, it can be seen that the aqueous arginine solution played an important role in protecting the stability of the rhFGF21-VPGXG$_{40}$ protein at 25 °C, and the protection effects of the aqueous arginine solutions with the three concentrations were not much different (Figure 15).

3. Determination of storage solutions for the rhFGF21-(VPGXG)$_{40}$ protein

The arginine (50 mmol/L) + citrate buffer solutions of pH 5.5 and pH 5.0 were prepared by dissolving arginine, citric acid, and sodium citrate in water. The method of replacement for the storage solutions of the rhFGF21-(VPGXG)$_{40}$ protein was as described in Section 1 of this Example, and the protein at the corresponding UV absorption peak was collected using a collector, i.e., the rhFGF21-(VPGXG)$_{40}$ protein replaced into the storage solution was obtained. Each group of rhFGF21-(VPGXG)$_{40}$ protein replaced into the protein storage solution was divided into two aliquots, one of which was stored in 25°C incubator away from light, and the other of which was stored in 4°C display cabinet away from light. Samples were taken at regular intervals for the 15% SDS-PAGE electrophoresis and HPLC assay (PBS buffer, 1 mL/min, chromatographic column: Agilent AdvanceBio SEC 300Å, 2.7 $\mu$m, 7.8×300 mm, Cat. No.: PL1180-5301), and the aqueous protein solution stored at -80°C was used as control.

The results showed that the protein preservation effect was improved when the arginine and citrate buffer solutions were mixed for protein preservation. When being preserved with citrate buffer solution only, the degradation of protein occurred after being left at 25°C for 14 days. When being preserved with arginine only, degradation occurred after being left at 25°C for 25 days. However, for the arginine + citrate buffer solutions, degradation of the protein occurred only after being stored for 35 days at 25°C in the arginine (50 mmol/L) + citrate buffer solution of pH 5.0, while no degradation was seen at this time in the arginine (50 mmol/L) + citrate buffer solution of pH 5.5. In order to determine the protective effect of the arginine + citrate buffer solution of pH 5.5 on the protein more precisely, it and the protein stored at -80°C were analyzed by HPLC (PBS buffer, 1 mL/min, chromatographic column: Agilent AdvanceBio SEC 300 Å, 2.7 $\mu$m, 7.8 × 300 mm, Cat. No.: PL1180-5301) and compared. The results showed that both were almost unchanged, indicating that storage of protein in the arginine + citrate buffer solution of pH 5.5 significantly improved the stability of the protein, and the physical and chemical stability of the protein was maintained during 35 days of storage (Figure 16). After the proteins were stored in the above two arginine + citrate buffer solutions at 4°C for 60 days, the results of SDS-PAGE electrophoresis of the two did not show significant differences, and neither showed the degraded bands. However, after HPLC assay (PBS buffer, 1 mL/min, chromatographic column: Agilent AdvanceBio SEC 300 Å, 2.7 $\mu$m, 7.8x300 mm, Cat. No.: PL1180-5301), it can be seen that only a small amount of degradation occurred in the protein preserved by the arginine + citrate buffer solution of pH 5.0 (i.e., the degraded bands were not separated by SDS-PAGE electrophoresis); while the protein preserved by the arginine + citrate buffer solution of pH 5.5 showed no degradation phenomenon after being detected by HPLC (PBS buffer, 1 mL/min, chromatographic column: Agilent AdvanceBio SEC 300 Å, 2.7 $\mu$m, 7.8 × 300 mm, Cat. No.: PL1180-5301) (Figure 17). It can be seen that the physical and chemical stability of the protein could be maintained relatively good by being preserved at 25 °C for 35 days or at 4 °C for 60 days using the above two arginine+citrate buffer solutions; relatively speaking, the arginine+citrate buffer solution of pH 5.5 could better maintain the physical and chemical stability of the protein.

4. Activity assay of the rhFGF21-(VPGXG)$_{40}$ protein in protein storage solution

The rhFGF21-(VPGXG)$_{40}$ proteins in the above storage solutions after 35 days of storage at -80 °C and 25 °C were added into HepG2 cells, and the cells were incubated at 37°C, 0.5% $CO_2$ for 24h, and then the remaining glucose content in the medium (DMEM, gibco, C11965500BT) was measured using the glucose assay kit (GOD-POD method).

The results showed that the protein stored at 25°C still had a good promotion effect on the glucose uptake of cells, and its promotion effect showed a dose-dependent relationship; compared with the corresponding doses (10nmol, 100nmol, 1000nmol) of the protein stored at -80°C, although the promotion effects of three doses (10nmol, 100nmol, 1000nmol) of the protein stored at 25°C all slightly decreased, differences therebetween were not significant, indicating that the proteins stored at 25°C in the above-mentioned storage solutions maintained good biological activity (Figure 18).

For the purposes of description and disclosure, all patents, patent applications, and other publications are hereby expressly incorporated by reference. These publications are provided only because their disclosures are earlier than the filing date of the present application. All statements regarding the dates of these documents or expressions regarding the contents of these documents are based on information available to the applicant, and do not constitute any admission of the correctness of the date of these documents or the contents of these documents. Besides, in any country, any reference to these publications herein does not constitute an admission that the publication is part of the common knowledge in the art.

Those skilled in the art will recognize that the scope of this application is not limited to various specific embodiments and examples described above, but various modifications, substitutions, or re-combinations can be made without departing from the spirit of this application, all of which fall within the protection scope of the present application.

**Claims**

1.  A recombinant human fibroblast growth factor 21 fusion protein, wherein the fusion protein comprises rhFGF21 and $(VPGXG)_n$ which are fused together, the rhFGF21 comprises an amino acid sequence set forth in SEQ ID NO: 1 or an amino acid sequence with at least 80% identity to SEQ ID NO: 1; X is A and/or V; and n is an integer selected from 20-80.

2.  The fusion protein of claim 1, wherein X is A and V, and the ratio of A to V is (1-3):(1-3), preferably 2:3; preferably, n is an integer selected from 40-60, preferably 40.

3.  The fusion protein of claim 1 or 2, wherein the fusion protein consists of rhFGF21, $(VPGXG)_n$, and an optional linker, which are fused together; preferably, an amino acid sequence of the linker is RS and/or GS; preferably, the fusion protein has an amino acid sequence set forth in SEQ ID NO: 2 or an amino acid sequence with at least 80% identity to SEQ ID NO: 2.

4.  A polynucleotide encoding the fusion protein of any of claims 1-3;

    preferably, a portion of the polynucleotide encoding rhFGF21 in the fusion protein comprises a nucleotide sequence set forth in SEQ ID NO: 3 or a nucleotide sequence with at least 80% identity to SEQ ID NO: 3; preferably, a portion of the polynucleotide encoding the linker in the fusion protein comprises a nucleotide sequence set forth in SEQ ID NO: 4 and/or SEQ ID NO: 5 or a nucleotide sequence with at least 80% identity to SEQ ID NO: 4 and/or SEQ ID NO: 5.

5.  The polynucleotide of claim 4, wherein the polynucleotide comprises a nucleotide sequence set forth in SEQ ID NO: 6 or a nucleotide sequence with at least 80% identity to SEQ ID NO: 6.

6.  A recombinant expression vector comprising the polynucleotide of claim 4 or 5;

    preferably, the recombinant expression vector is a prokaryotic recombinant expression vector; more preferably, the recombinant expression vector is a prokaryotic recombinant expression vector carrying a molecular chaperone; further preferably, the molecular chaperone is a small molecule ubiquitin-like modified protein; or further preferably, the recombinant expression vector is a recombinant pSUMO vector or recombinant PET series of vectors.

7.  A recombinant cell comprising the recombinant expression vector of claim 6 or having the polynucleotide of claim 4 or 5 integrated in its genome; preferably, a host cell for constructing the recombinant cell is a prokaryotic host cell;

    more preferably, the prokaryotic host cell is selected from *E. coli, Agrobacterium tumefaciens, Staphylococcus aureus, Staphylococcus albus,*
    *Lactobacillus acidophilus, Bacillus anthracis, Bacillus subtilis* or *Bacillus thuringiensis.*

8.  A pharmaceutical composition comprising the fusion protein of any of claims 1-3;
    preferably, the pharmaceutical composition further comprises a pharmaceutically acceptable excipient.

9.  A kit comprising the fusion protein of any of claims 1-3 or the pharmaceutical composition of claim 6;

    preferably, the kit further comprises a citrate buffer solution of pH 5.0-5.5; preferably, the kit further comprises an aqueous arginine solution, preferably an aqueous arginine solution of 50 mmol/L-150 mmol/L; preferably, the kit further comprises an arginine + citrate buffer solution of pH 5.0-5.5; preferably, the kit further comprises an aqueous arginine solution and a citrate buffer solution of pH 5.0-5.5, and the fusion protein or the pharmaceutical composition, the citrate buffer solution of pH 5.0-5.5, and the aqueous arginine solution are packaged together or separately.

10. The fusion protein of any of claims 1-3 for use in the prevention or treatment of a disease associated with abnormal glucose metabolism; preferably, the disease associated with abnormal glucose metabolism is selected from diabetes mellitus and diabetes-related metabolic disease, more preferably, the diabetes-related metabolic disease is selected from diabetic nephropathy, dyslipidemia, obesity, cardiovascular diseases, metabolic syndrome, lipid metabolism disorders, non-alcoholic fatty liver diseases, or nervous system diseases.

**11.** A method of producing the fusion protein of any of claims 1-3, comprising:

culturing the recombinant cell of claim 7, then inducing the recombinant cell to express and purifying an expression product to obtain the fusion protein;
preferably, the expression of the recombinant cell is induced using an inducer selected from IPTG and/or lactose;
preferably, the purification of the expression product comprises: (i) after being induced to express, centrifuging the recombinant cell to collect bacteria,
collecting a supernatant by centrifugation after breaking the bacteria; (ii) subjecting the supernatant to ion exchange chromatography and collecting an eluate; (iii) subjecting the eluate to affinity chromatography to obtain the fusion protein.

**12.** The method of claim 11, further comprising:

(1) synthesizing a polynucleotide encoding rhFGF21 and synthesizing a polynucleotide encoding $(VPGXG)_{20}$;
(2) performing enzyme digestion and ligation to the polynucleotide encoding rhFGF21 and the polynucleotide encoding $(VPGXG)_{20}$, and obtaining a polynucleotide encoding the fusion protein through PCR reaction;
(3) performing enzyme digestion and ligation to the polynucleotide encoding the fusion protein and an expression vector to obtain a recombinant expression vector, introducing the recombinant expression vector into a host cell, and screening to obtain the recombinant cell.

**13.** The method of claim 12, in step (1), the polynucleotide sequence encoding rhFGF21 comprises a nucleotide sequence set forth in SEQ ID NO: 3 or a nucleotide sequence with at least 80% identity to SEQ ID NO: 3;
preferably, in step (1), the polynucleotide encoding $(VPGXG)_{20}$ comprises a nucleotide sequence set forth in SEQ ID NO: 7 or a nucleotide sequence with at least 80% identity to SEQ ID NO: 7.

**14.** The method of claim 12 or 13, in step (2), a polynucleotide encoding a linker selected from RS and/or GS is optionally attached to 5' end and 3' end of the polynucleotide encoding $(VPGXG)_{20}$, respectively;
preferably, in step (2), the enzyme digestion is performed using *Bg*/II restriction endonuclease and *Bam*HI restriction endonuclease.

**15.** The method of any of claims 12-14, in step (3), the enzyme digestion is performed using *Bsa*I restriction endonuclease and *Bam*HI restriction endonuclease;

preferably, in step (3), the expression vector is selected from pSUMO vector and PET series of vectors; and
preferably, in step (3), the expression vector is pSUMO vector and the host cell is *E. coli Rossetta* (DE3).

Fig. 1

Fig. 2

Fig. 3

after 12 hours

Fig. 4

23

Fig. 5

Fig. 6

Fig. 8

Fig. 9

Fig. 10

Fig. 11

Fig. 12

normal control group     model control group     rhFGF21-VPGXG$_{40}$

Fig. 13

Fig. 14

Fig. 15

Fig. 16

Fig. 17

Fig. 18

## INTERNATIONAL SEARCH REPORT

| International application No. |
|---|
| **PCT/CN2021/096292** |

**A. CLASSIFICATION OF SUBJECT MATTER**

C07K 16/22(2006.01)i; C07K 14/50(2006.01)i; A61K 38/00(2006.01)i; A61P 3/10(2006.01)i

According to International Patent Classification (IPC) or to both national classification and IPC

**B. FIELDS SEARCHED**

Minimum documentation searched (classification system followed by classification symbols)

C07K, A61K, A61P

Documentation searched other than minimum documentation to the extent that such documents are included in the fields searched

Electronic data base consulted during the international search (name of data base and, where practicable, search terms used)

DWPI; CNABS; CNTXT; USTXT; WOTXT; PUBMED; ISI WEB OF SCIENCE; CNKI: 成纤维细胞生长因子, 重组, 融合蛋白, 弹性蛋白样肽, 人源化, 血糖, 血脂, 代谢, FGF21, rhFGF21, ELP, VPGXG, humanization, fusion protein, recombination, blood glucose, blood lipid, metabolism, SEQ ID NO: 1-3

**C. DOCUMENTS CONSIDERED TO BE RELEVANT**

| Category* | Citation of document, with indication, where appropriate, of the relevant passages | Relevant to claim No. |
|---|---|---|
| X | CN 109310641 A (PHASEBIO PHARMACEUTICALS, INC.) 05 February 2019 (2019-02-05) abstract, claims 1, 6, 10, description paragraphs 25-28, 33, 50-58, 186-191, embodiments 1, 6 | 1-15 |
| X | WO 2018018010 A1 (METACRINE, INC.) 25 January 2018 (2018-01-25) abstract, paragraphs 31-32, 299 | 1-15 |
| X | GILROY, C.A. et al. "Fusion of fibroblast growth factor 21 to a thermally responsive biopolymer forms an injectable depot with sustained anti-diabetic action" *JOURNAL OF CONTROLLED RELEASE*, Vol. 277, 10 May 2018 (2018-05-10), pp. 154-164 | 1-15 |
| A | CN 110343183 A (DIANDOU GENE TECHNOLOGY (NANJING) CO., LTD.) 18 October 2019 (2019-10-18) entire document | 1-15 |
| A | WO 2019118701 A1 (PURDUE RESEARCH FOUNDATION) 20 June 2019 (2019-06-20) entire document | 1-15 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **11 August 2021** | **26 August 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/096292**

### C. DOCUMENTS CONSIDERED TO BE RELEVANT

| Category* | Citation of document, with indication, where appropriate, of the relevant passages | Relevant to claim No. |
|---|---|---|
| A | WO 2019236769 A1 (UNIVERSITY OF MISSISSIPPI MEDICAL CENTER) 12 December 2019 (2019-12-12)<br>entire document | 1-15 |
| A | 龚琦 等 (GONG, Qi et al.). "FGF21的代谢调控及临床应用 (Metabolic Actions of FGF21 and Its Clinical Outcomes)"<br>中国细胞生物学学报 (Chinese Journal of Cell Biology),<br>Vol. 37, No. 4, 26 March 2015 (2015-03-26),<br>pp. 460-468 | 1-15 |

Form PCT/ISA/210 (second sheet) (January 2015)

## INTERNATIONAL SEARCH REPORT

International application No.

**PCT/CN2021/096292**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

INTERNATIONAL SEARCH REPORT
Information on patent family members

International application No.

**PCT/CN2021/096292**

| Patent document cited in search report | | | Publication date (day/month/year) | Patent family member(s) | | | Publication date (day/month/year) |
|---|---|---|---|---|---|---|---|
| CN | 109310641 | A | 05 February 2019 | EP | 3452021 | A1 | 13 March 2019 |
| | | | | WO | 2017192449 | A1 | 09 November 2017 |
| | | | | JP | 2019514949 | A | 06 June 2019 |
| | | | | KR | 20190005171 | A | 15 January 2019 |
| | | | | MX | 2018013546 | A | 22 April 2019 |
| | | | | EP | 3452021 | A4 | 15 January 2020 |
| | | | | US | 2019218274 | A1 | 18 July 2019 |
| | | | | BR | 112018072783 | A2 | 12 March 2019 |
| | | | | CA | 3021644 | A1 | 09 November 2017 |
| | | | | AU | 2017261216 | A1 | 15 November 2018 |
| WO | 2018018010 | A1 | 25 January 2018 | None | | | |
| CN | 110343183 | A | 18 October 2019 | None | | | |
| WO | 2019118701 | A1 | 20 June 2019 | None | | | |
| WO | 2019236769 | A1 | 12 December 2019 | US | 2019365922 | A1 | 05 December 2019 |
| | | | | EP | 3801600 | A1 | 14 April 2021 |

Form PCT/ISA/210 (patent family annex) (January 2015)

## REFERENCES CITED IN THE DESCRIPTION

**Non-patent literature cited in the description**

- **SINGLETON et al.** Dictionary of Microbiology and Molecular Biology. J. Wiley & Sons, 1994
- **SAMBROOK et al.** Molecular Cloning, A Laboratory Manual. Cold Springs Harbor Press, 1989
- **R.C. ROWE et al.** Handbook of Pharmaceutical Excipients. Chemical Industry Press, 2005

- Manufacture and Application of Microbial Medium. China Agricultural Press, 1995
- Practical Handbook of Microbial Medium. Jilin Science and Technology Press, 2005
- Molecular Cloning: A Laboratory Manual. Science Press, 2017